# EUROPEAN PATENT SPECIFICATION

(11) **EP 3 917 550 B1**
(45) Date of publication and mention of the grant of the patent: **24.01.2024**
(21) Application number: 20725524.1
(22) Date of filing: 11.05.2020
(51) Int. Cl.: A61K 35/74, A61P 25/00

(54) **COMPOSITIONS COMPRISING BLAUTIA HYDROGENOTROPHICA IN THE TREATEMNT OF FIBROMYALGIA**
ZUSAMMENSETZUNGEN MIT BLAUTIA HYDROGENOTROPHICA ZUR BEHANDLUNG VON FIBROMYALGIE
COMPOSITIONS COMPRENANT DES BLAUTIA HYDROGENOTROPHICA DANS LE TRAITEMENT DE FIBROMYALGIE

(30) Priority: 10.05.2019 EP 19173945
(43) Date of publication of application: 08.12.2021
(73) Proprietor: CJ Bioscience, Inc., Jung-gu Seoul 04527 (KR)
(72) Inventor: STEVENSON, Alex, Aberdeen Aberdeenshire AB25 2ZS (GB); CHETAL, Sasha, Aberdeen Aberdeenshire AB25 2ZS (GB)
(74) Representative: Bates, Philip Ian
(86) International application number: PCT/EP2020/063084
(87) International publication number: WO 2020/229428

(56) References cited:
- WO-A1-2017/160711
- WO-A1-2019/046646
- WO-A2-2014/121298
- US-A1- 2016 271 188
- US-A1- 2019 134 106

## Description

### TECHNICAL FIELD

This invention is in the field of compositions comprising bacterial strains isolated from the mammalian digestive tract and the use of such compositions in the treatment of disease.

### BACKGROUND TO THE INVENTION

The human intestine is thought to be sterile *in utero,* but it is exposed to a large variety of maternal and environmental microbes immediately after birth. Thereafter, a dynamic period of microbial colonization and succession occurs, which is influenced by factors such as delivery mode, environment, diet and host genotype, all of which impact upon the composition of the gut microbiota, particularly during early life. Subsequently, the microbiota stabilizes and becomes adult-like [1]. The human gut microbiota contains more than 1,500 different phylotypes dominated in abundance levels by two major bacterial divisions *(phyla),* the Bacteroidetes and the Firmicutes [2-3]. The successful symbiotic relationships arising from bacterial colonization of the human gut have yielded a wide variety of metabolic, structural, protective and other beneficial functions. The enhanced metabolic activities of the colonized gut ensure that otherwise indigestible dietary components are degraded with release of by-products providing an important nutrient source for the host and additional health benefits. Similarly, the immunological importance of the gut microbiota is well-recognized and is exemplified in germ-free animals which have an impaired immune system that is functionally reconstituted following the introduction of commensal bacteria [4-6].

Dramatic changes in microbiota composition have been documented in gastrointestinal disorders such as inflammatory bowel disease (IBD). For example, the levels of *Clostridium* cluster XIVa and *Clostridium* cluster XI *(F. prausnitzii)* bacteria are reduced in IBD patients whilst numbers of *E. coli* are increased, suggesting a shift in the balance of symbionts and pathobionts within the gut [7-11].

In recognition of the potential positive effect that certain bacterial strains may have on the animal gut, various strains have been proposed for use in the treatment of various diseases (see, for example, [12-15]). A number of strains, including mostly *Lactobacillus* and *Bifidobacterium* strains, have been proposed for use in treating various bowel disorders (see [16] for a review). Strains of the genus *Blautia* have also been proposed for use in modulating the microbial balance of the digestive ecosystem [17]. However, the relationship between different bacterial strains and different diseases, and the precise effects of particular bacterial strains on the gut and at a systemic level and on any particular types of diseases, are poorly characterised. US 2016/271188 discloses probiotic and prebiotic compositions, and methods of use thereof for treatment of gastrointestinal disorders. WO 2014/121298 discloses methods of populating a gastrointestinal tract. WO 2017/160711 discloses modulation of the gut microbiome to treat mental disorders or diseases of the central nervous system. US 2019/134106 discloses compositions for fecal floral transplantation and methods for making and using them and device for delivering them. WO 2019/046646 discloses methods and compositions for treatment of microbiome-associated disorders. Coyne et al (2017) Postgraduate Medicine, 129, 1, 22-31 discusses how to discriminate between neuropathic pain and sensory hypersensitivity using the chronic pain questions (CPQ) and Stanos et al. (2016) Postgraduate Medicine, 128, 5, 502-515 discusses rethinking chronic pain in a primary care setting.

There is a requirement for the potential effects of gut bacteria to be characterised so that new therapies using bacteria can be developed.

### SUMMARY OF THE INVENTION

The inventors have developed new therapies for treating and preventing sensory hypersensitivity, allodynia and/or hyperalgesia. In particular, the invention provides a composition comprising a bacterial strain of the species *Blautia hydrogenotrophica* for use in a method of treating or preventing sensory hypersensitivity in a subject diagnosed with fibromyalgia.

Sensory hypersensitivity is frequently associated with allodynia and/or hyperalgesia and the inventors have identified that bacterial strains from the genus *Blautia* can be effective for reducing allodynia and/or hyperalgesia. Therefore, in one embodiment, the invention provides a composition comprising a bacterial strain of the species *Blautia hydrogenotrophica,* for use in a method of treating or preventing allodynia and/or hyperalgesia which are both associated with sensory hypersensitivity in a subject diagnosed with fibromyalgia. As described in the examples, oral administration of compositions comprising *Blautia hydrogenotrophica* may reduce allodynia in an animal model of fibromyalgia. Therefore, the invention also provides a composition comprising a bacterial strain of the species *Blautia hydrogenotrophica* for use in a method of treating or preventing fibromyalgia.

The use of organisms from the genus *Blautia* to treat visceral hypersensitivity has been disclosed in International Patent Publication No. WO2017/148596. However, as explained in more detail below, visceral hypersensitivity and allodynia/hyperalgesia have different pathophysiologies and causes. Accordingly, that publication does not suggest that such organisms can be successfully used to treat allodynia and/or hyperalgesia.

The inventors have shown that the compositions of the invention work particularly well in a model of fibromyalgia.

The bacterial strain in the composition is *Blautia hydrogenotrophica.* Preferably, the bacterial strain has a 16s rRNA sequence that is at least 95%, 96%, 97%, 98%, 99%, 99.5% or 99.9% identical to SEQ ID NO:5. Most preferably, the bacterial strain in the composition is the *Blautia hydrogenotrophica* strain deposited under accession number DSM 14294. Preferably, the bacterial strain for use in the invention has the 16s rRNA sequence represented by SEQ ID NO:5. In other embodiments, the bacterial strain in the composition has a 16s rRNA sequence that is at least 95%, 96%, 97%, 98%, 99%, 99.5% or 99.9% identical to SEQ ID NO: 5.

In certain embodiments, the composition of the invention is for oral administration. Oral administration is convenient for patients and practitioners and allows delivery to and/or partial or total colonisation of the intestine.

In certain embodiments, the composition of the invention comprises one or more pharmaceutically acceptable excipients or carriers.

In certain embodiments, the composition of the invention has been lyophilised. The composition of the invention can also comprise a lyophilised bacteria strain of the species *Blautia hydrogenotrophica.* Lyophilisation is an effective and convenient technique for preparing stable compositions that allow delivery of bacteria, and is shown to provide effective compositions in the examples.

### BRIEF DESCRIPTION OF DRAWINGS

**Figure 1****:** Weight gain over time. Error bars denote SEM.
**Figure 2****:** Somatosensory responses (hindpaw placing). Asterisks indicate statistically significant differences from the appropriate contralateral response. Error bars denote SEM. Normal Control N = 12, Disease Control N = 24, *Blautia hydrogenotrophica* N = 12, Lyobuffer N = 12.
**Figure 3****:** Mechanical allodynia response (50% paw withdrawal threshold) in rat model of fibromyalgia. Error bars denote SEM. N = 12 per group.
**Figure 4****:** Muscle compression withdrawal threshold testing. Error bars denote SEM. N = 12 per group.

### DISCLOSURE OF THE INVENTION

### Bacterial strains

The compositions of the invention comprise a bacterial strain of the species *Blautia hydrogenotrophica.* The examples demonstrate that bacteria of this genus are useful for treating or preventing sensory hypersensitivity and are particularly useful for treating or preventing allodynia and/or hyperalgesia.

Examples of *Blautia* strains include *Blautia hydrogenotrophica, B. stercoris, B. faecis, B. coccoides, B. glucerasea, B. hansenii, B. luti, B. producta, B. schinkii* and *B. wexlerae.* The *Blautia* species are Gram-reaction-positive, non-motile, non-spore forming bacteria that may be either coccoid or oval and all are obligate anaerobes that produce acetic acid as the major end-product of glucose fermentation [18]. *Blautia* may be isolated from the human gut, although *B. producta* was isolated from a septicaemia sample.

*Blautia hydrogenotrophica* (previously known as *Ruminococcus hydrogenotrophicus*) has been isolated from the guts of mammals, is strictly anaerobic, and metabolises H₂/CO₂ to acetate, which may be important for human nutrition and health. The type strain of *Blautia hydrogenotrophica* is S5a33 = DSM 10507 = JCM 14656. The GenBank accession number for the 16S rRNA gene sequence of *Blautia hydrogenotrophica* strain S5a36 is X95624.1 (disclosed herein as SEQ ID NO:5). This exemplary *Blautia hydrogenotrophica* strain is described in [18] and [19]. The S5a33 strain and the S5a36 strain correspond to two subclones of a strain isolated from a faecal sample of a healthy subject. They show identical morphology, physiology and metabolism and have identical 16S rRNA sequences. Thus, in some embodiments, the *Blautia hydrogenotrophica* for use in the invention has the 16S rRNA sequence of SEQ ID NO:5.

The *Blautia hydrogenotrophica* bacterium deposited under accession number DSM 14294 was tested in the examples and is also referred to herein as strain BH. Strain BH was deposited with the Deutsche Sammlung von Mikroorganismen [German Microorganism Collection] (Mascheroder Weg 1b, 38124 Braunschweig, Germany) in January 1996 as *"Ruminococcus hydrogenotrophicus"* under accession number DSM 14294 as "S5a33" on 10th May 2001. The depositor was INRA Laboratoire de Microbiologie CR de Clermont-Ferrand/Theix 63122 Saint Genès Champanelle, France. Ownership of the deposits has passed to 4D Pharma Plc by way of assignment.

The GenBank accession number for the 16S rRNA gene sequence of *Blautia stercoris* strain GAM6-1^{T} is HM626177 (disclosed herein as SEQ ID NO:1). An exemplary *Blautia stercoris* strain is described in [20]. The type strain of *Blautia wexlerae* is WAL 14507 = ATCC BAA-1564 = DSM 19850 [18]. The GenBank accession number for the 16S rRNA gene sequence of *Blautia wexlerae* strain WAL 14507 T is EF036467 (disclosed herein as SEQ ID NO:2). This exemplary *Blautia wexlerae* strain is described in [18].

A preferred *Blautia stercoris* strain is the strain deposited under accession number NCIMB 42381, which is also referred to herein as strain 830. A 16S rRNA sequence for the 830 strain is provided in SEQ ID NO:3. Strain 830 was deposited with the international depositary authority NCΠVIB, Ltd. (Ferguson Building, Craibstone Estate, Bucksburn, Aberdeen, AB21 9YA, Scotland) by GT Biologies Ltd. (Life Sciences Innovation Building, Aberdeen, AB25 2ZS, Scotland) on 12th March 2015 as *"Blautia stercoris* 830" and was assigned accession number NCIMB 42381. GT Biologies Ltd. subsequently changed its name to 4D Pharma Research Limited.

A preferred *Blautia wexlerae* strain is the strain deposited under accession number NCIMB 42486. A 16S rRNA sequence for this strain is provided in SEQ ID NO:4. The strain was deposited with the international depositary authority NCIMB, Ltd. (Ferguson Building, Craibstone Estate, Bucksburn, Aberdeen, AB21 9YA, Scotland) by 4D Pharma Research Ltd. (Life Sciences Innovation Building, Aberdeen, AB25 2ZS, Scotland) on 16th November 2015 as *"Blautia*/*Ruminococcus"* and was assigned accession number NCIMB 42486.

A preferred *Blautia producta* strain for use in the invention is the strain deposited under accession number NCIMB 43170 with international depositary authority NCΠVIB, Ltd. (Ferguson Building, Craibstone Estate, Bucksburn, Aberdeen, AB21 9YA, Scotland) by 4D Pharma Research Limited (Life Sciences Innovation Building, Aberdeen, AB25 2ZS, Scotland) on 20th August 2018 as *"Blautia producta".*

Bacterial strains closely related to the strain tested in the examples are also expected to be effective for treating or preventing allodynia and/or hyperalgesia. Preferably, the bacterial strain for use in the invention has a 16s rRNA sequence that is at least 95%, 96%, 97%, 98%, 99%, 99.5% or 99.9% identical to SEQ ID NO:5.

Bacterial strains that are biotypes of the bacterium deposited under accession number DSM 14294 or biotypes of the bacteria deposited under accession numbers NCIMB 42381, NCΠVIB 42486, and NCΠVIB 43170 are also expected to be effective for treating or preventing allodynia and/or hyperalgesia. A biotype is a closely related strain that has the same or very similar physiological and biochemical characteristics.

Strains that are biotypes of a bacterium deposited under accession number DSM 14294, NCIMB 42381, NCIMB 42486, or NCΠVVIB 43170 and that are suitable for use in the invention may be identified by sequencing other nucleotide sequences for a bacterium deposited under accession number DSM 14294, NCINM 42381, NCIMB 42486 or NCIMB 43170. For example, substantially the whole genome may be sequenced and a biotype strain for use in the invention may have at least 95%, 96%, 97%, 98%, 99%, 99.5% or 99.9% sequence identity across at least 80% of its whole genome (e.g. across at least 85%, 90%, 95% or 99%, or across its whole genome). For example, in some embodiments, a biotype strain has at least 98% sequence identity across at least 98% of its genome or at least 99% sequence identity across 99% of its genome. Other suitable sequences for use in identifying biotype strains may include hsp60 or repetitive sequences such as BOX, ERIC, (GTG)5, or REP [21]. Biotype strains may have sequences with at least 95%, 96%, 97%, 98%, 99%, 99.5% or 99.9% sequence identity to the corresponding sequence of a bacterium deposited under accession number DSM 14294, NCIMB 42381, NCIMB 42486, or NCIMB 43170. In some embodiments, a biotype strain has a sequence with at least 97%, 98%, 99%, 99.5% or 99.9% sequence identity to the 16S rRNA sequence of SEQ ID NO:5.

Alternatively, strains that are biotypes of a bacterium deposited under accession number DSM 14294, NCΠVIB 42381, NCΠVIB 42486 or NCIMB 43170 and that are suitable for use in the invention may be identified by using the accession number DSM 14294 deposit, the accession number NCIMB 42381 deposit, the accession number NCIMB 42486 deposit, or the accession number NCIMB 43170 deposit, and restriction fragment analysis and/or PCR analysis, for example by using fluorescent amplified fragment length polymorphism (FAFLP) and repetitive DNA element (rep)-PCR fingerprinting, or protein profiling, or partial 16S or 23s rDNA sequencing. In preferred embodiments, such techniques may be used to identify other *Blautia hydrogenotrophica, Blautia stercoris* or *Blautia wexlerae* strains.

In certain embodiments, strains that are biotypes of a bacterium deposited under accession number DSM 14294, NCIMB 42381, NCΠVVIB 42486, or NCΠVVIB 43170 and that are suitable for use in the invention are strains that provide the same pattern as a bacterium deposited under accession number DSM 14294, NCΠVIB 42381, NCΠVVIB 42486 or NCΠVIB 43170 when analysed by amplified ribosomal DNA restriction analysis (ARDRA), for example when using Sau3AI restriction enzyme (for exemplary methods and guidance see, for example [22]). Alternatively, biotype strains are identified as strains that have the same carbohydrate fermentation patterns as a bacterium deposited under accession number DSM 14294, NCΠVVIB 42381, NCΠVVIB 42486 or NCIMB 43170.

Other *Blautia* strains that are useful in the invention, such as biotypes of a bacterium deposited under accession number DSM 14294, NCΠVVIB 42381 NCΠVVIB 42486, or NCΠVVIB 43170 may be identified using any appropriate method or strategy, including the assays described in the examples. For instance, strains for use in the invention may be identified by culturing bacteria and administering them to rats before testing in the Von Frey microfilament assay. In particular, bacterial strains that have similar growth patterns, metabolic type and/or surface antigens to a bacterium deposited under accession number DSM 14294, NCIMB 42381, NCIMB 42486 or NCIMB 43170 may be useful in the invention. A useful strain will have comparable activity to the DSM 14294, NCΠVVIB 42381, NCΠVVIB 42486 or NCΠVIB 43170 strain as assessed, for example, by the Von Frey microfilament assay. In particular, a biotype strain will elicit comparable effects on the fibromyalgia model to the effects shown in the examples, which may be identified by using the culturing and administration protocols described in the examples.

A particularly preferred strain of the invention is the *Blautia hydrogenotrophica* strain deposited under accession number DSM 14294. This is the exemplary strain tested in the examples and shown to be effective for treating disease.

A derivative of the strain deposited under accession number DSM 14294, NCIMB 42381, NCIMB 42486, or NCIMB 43170 may be a daughter strain (progeny) or a strain cultured (subcloned) from the original. A derivative of a strain of the invention may be modified, for example at the genetic level, without ablating the biological activity. In particular, a derivative strain of the invention is therapeutically active. A derivative strain will have comparable microbiota modulatory activity to the original DSM 14294, NCIMB 42381, NCIMB 42486, NCIMB 43170 strain. In particular, a derivative strain will elicit comparable effects on the allodynia and/or hyperalgesia model to the effects shown in the examples, which may be identified by using the culturing and administration protocols described in the examples. A derivative of the DSM 14294 strain will generally be a biotype of the DSM 14294 strain. A derivative of the NCIMB 42381 strain will generally be a biotype of the NCIMB 42381 strain. A derivative of the NCIMB 42486 strain will generally be a biotype of the NCIMB 42486 strain. A derivative of the NCIMB 43170 strain will generally be a biotype of the NCIMB 43170 strain.

The bacterial strain may also be a strain that has the same safety and therapeutic efficacy characteristics as the strains deposited under accession number DSM 14294, NCΠVVIB 42381, NCΠVVIB 42486 or NCΠVIB 43170 and such cells are encompassed by the invention.

In preferred embodiments, the bacterial strains in the compositions of the invention are viable and capable of partially or totally colonising the intestine.

### Therapeutic uses

The compositions of the invention may be for use in treating sensory hypersensitivity. They may be used for treating allodynia and/or hyperalgesia. Preferably, the compositions of the invention are for use in treating allodynia associated with fibromyalgia, as the inventors have seen good effects for this, as shown in the examples.

There are three main types of pain pathophysiology-nociceptive, neuropathic and sensory hypersensitivity. Sensory hypersensitivity describes a type of pain that exists without an identifiable nerve or tissue damage. This type of pain is thought to be a result of persistent dysfunction of neurons throughout the CNS that leads to lowering of pain thresholds and amplification of sensory signals. It is referred to by many names including centralized, dysfunctional, or idiopathic pain, central sensitization, and central sensitivity syndromes. The hallmark of this type of pain appears to be generalized hypersensitivity to a variety of stimuli including mechanical, thermal, olfactory, auditory, and visual cues. Sensory hypersensitivity conditions include: Fibromyalgia, Irritable bowel syndrome, Tension-type headaches, Interstitial cystitis/pelvic pain syndrome, Tempo-mandibular joint disorder, Chronic fatigue syndrome, Restless leg syndrome and Neck and back pain without structural pathology [23].

Patients suffering from neuropathic pain and sensory hypersensitivity can be discriminated between using the Chronic Pain Questions according to ref [24] and the severity of the sensory hypersensitivity can be assessed using the Sensory Hypersensitivity Scale described in ref [25].

Allodynia (pain resulting from a non-painful stimulus) and hyperalgesia (increased sensitivity to pain) are commonly associated with sensory hypersensitivity, a term used to describe pain in the absence of identifiable damage to nerves or other tissues. Allodynia and hyperalgesia are hypothesized to be the result of persistent neuronal dysregulation or dysfunction. These are frequently associated with a variety of conditions, including neuropathy, complex regional pain syndrome, postherpetic neuralgia, fibromyalgia, or migraine. The compositions of the invention can be used to treat a subject diagnosed with one or more of these conditions.

In a preferred embodiment, the compositions of the invention are for use in treating a subject diagnosed with fibromyalgia, in particular for treating a subject diagnosed with allodynia associated with fibromyalgia. These embodiments are preferred because the inventors have seen good effects with the compositions of the invention in a rat model of fibromyalgia. The examples demonstrate the ability of compositions of the invention to have beneficial effects on several factors that are conducive to promoting a more positive outcome in a rat model of fibromyalgia. These include increased weight gain, improved performance in somatosensory testing and a reduction in mechanical allodynia.

Fibromyalgia is generally believed to be the result of sensory hypersensitivity, a term used to describe pain in the absence of identifiable damage to nerves or other tissues. This type of pain is hypothesized to be the result of persistent neuronal dysregulation or dysfunction. As a result of the sensory hypersensitivity, patients experience allodynia and hyperalgesia. Fibromyalgia is generally characterized by chronic pain and fatigue as well as sensitivity/tenderness in the joints and muscle tissue and many people with fibromyalgia also report sensitivity to light, headaches and migraine attacks as symptoms or co-morbidities of the condition.

In contrast to sensory hypersensitivity, visceral hypersensitivity is the term used to describe the experience of pain within the inner organs at a level that is more intense than normal. This type of pain is related to the damage of somatic or visceral tissue, due to trauma or inflammation, and is known as nociceptive pain. Nociceptive pain originates from outside of the nervous system and the nerve cells transmitting the pain impulses are believed to be functioning normally. Interestingly, visceral hypersensitivity is a hallmark characteristic of Irritable Bowel Syndrome (IBS). While IBS is sometimes found in sufferers of fibromyalgia, it has been shown, for example in reference [26], that fibromyalgia patients do not usually suffer from visceral hypersensitivity. In other words, fibromyalgia and visceral hypersensitivity are discrete components of different conditions with different causes and pathophysiologies.

As demonstrated in the examples, bacterial compositions of the invention may be effective for reducing allodynia and/or hyperalgesia, in particular in a patient diagnosed with fibromyalgia. In embodiments, the patient does not suffer from visceral hypersensitivity. In this respect, "suffer" means that a patient has either been formally diagnosed at the time of treatment or a patient who has not yet received a formal diagnosis but who experiences symptoms of the disease.

The compositions of the invention may be for use in treating or preventing allodynia and/or hyperalgesia in a subject diagnosed with neuropathy, complex regional pain syndrome, postherpetic neuralgia, fibromyalgia, or migraine. In preferred embodiments, the compositions of the invention are for use in treating or preventing allodynia and/or hyperalgesia in a subject diagnosed with fibromyalgia.

Treatment or prevention of allodynia and/or hyperalgesia may refer to, for example, an alleviation of the severity of symptoms or a reduction in the frequency of exacerbations or the range of triggers that are a problem for the patient. For example, in some embodiments the composition of the invention is for use in treating or preventing severe allodynia and/or hyperalgesia. The subject having severe allodynia and/or hyperalgesia may be a subject diagnosed with neuropathy, complex regional pain syndrome, postherpetic neuralgia, fibromyalgia, or migraine. In some embodiments the subject having severe visceral hypersensitivity is a subject diagnosed with fibromyalgia.

In some embodiments, the sensory hypersensitivity (e.g. the allodynia and/or hyperalgesia) is not caused by an autoimmune disease. Furthermore, in some embodiments the subject having sensory hypersensitivity (e.g. severe allodynia and/or hyperalgesia) is a subject diagnosed with neuropathy, wherein the neuropathy is not caused by an autoimmune disease.

### Modes of administration

Preferably, the compositions of the invention are formulated to be administered to the gastrointestinal tract in order to enable delivery to and/or partial or total colonisation of the intestine with the bacterial strain of the invention. In some embodiments, the term "total colonisation of the intestine" means that bacteria have colonised all parts of the intestine (i.e. the small intestine, large intestine and rectum). In further embodiments of the invention, the term "total colonisation" or "partial colonisation" means that the bacteria are retained permanently or temporarily in the intestine, respectively. Generally, the compositions of the invention are administered orally, but they may be administered rectally, intranasally, or via buccal or sublingual routes.

In certain embodiments, the compositions of the invention may be administered as a foam, as a spray or a gel.

In certain embodiments, the compositions of the invention may be administered as a suppository, such as a rectal suppository, for example in the form of a theobroma oil (cocoa butter), synthetic hard fat (e.g. suppocire, witepsol), glycero-gelatin, polyethylene glycol, or soap glycerin composition.

In certain embodiments, the compositions of the invention are administered to the gastrointestinal tract via a tube, such as a nasogastric tube, orogastric tube, gastric tube, jejunostomy tube (J tube), percutaneous endoscopic gastrostomy (PEG), or a port, such as a chest wall port that provides access to the stomach, jejunum and other suitable access ports.

The compositions of the invention may be administered once, or they may be administered sequentially as part of a treatment regimen. In certain embodiments, the compositions of the invention are to be administered daily (either once or several times).

In certain embodiments, the compositions of the invention are administered regularly, such as daily, every two days, or weekly, for an extended period of time, such as for at least one week, two weeks, one month, two months, six months, or one year.

In some embodiments the compositions of the invention are administered for 7 days, 14 days, 16 days, 21 days or 28 days or no more than 7 days, 14 days, 16 days, 21 days or 28 days. For example, in some embodiments the compositions of the invention are administered for 16 days.

In certain embodiments of the invention, treatment according to the invention is accompanied by assessment of the patient's gut microbiota. Treatment may be repeated if delivery of and/or partial or total colonisation with the strain of the invention is not achieved such that efficacy is not observed, or treatment may be ceased if delivery and/or partial or total colonisation is successful, and efficacy is observed.

In certain embodiments, the composition of the invention may be administered to a pregnant animal, for example a mammal such as a human in order to prevent allodynia and/or hyperalgesia developing in her child *in utero* and/or after it is born.

The compositions of the invention may be administered to a patient that has been diagnosed with sensory hypersensitivity. They may also be administered to a patient that has been diagnosed with allodynia and/or hyperalgesia or a disease or condition associated with allodynia and/or hyperalgesia (in particular fibromyalgia), or that has been identified as being at risk of allodynia and/or hyperalgesia. The compositions may also be administered as a prophylactic measure to prevent the development of allodynia and/or hyperalgesia in a healthy patient.

The compositions of the invention may be administered to a patient that has been identified as having an abnormal gut microbiota. For example, the patient may have reduced or absent colonisation by *Blautia,* and in particular *Blautia hydrogenotrophica, Blautia stercoris, Blautia wexlerae, Blautia producta* or *Blautia coccoides.*

The compositions of the invention may be administered as a food product, such as a nutritional supplement.

Generally, the compositions of the invention are for the prevention or treatment of humans, although they may be used to treat animals including monogastric mammals such as poultry, pigs, cats, dogs, horses or rabbits. The compositions of the invention may be useful for enhancing the growth and performance of animals. If administered to animals, oral gavage may be used.

In some embodiments, the subject to whom the composition is to be administered is an adult human. In some embodiments, the subject to whom the composition is to be administered is an infant human.

### Compositions

The compositions of the invention comprise bacteria. The inventors have identified the surprising ability of bacteria from the genus *Blautia* to treat or prevent sensory hypersensitivity. However, in order for bacteria from the genus *Blautia* to exert their beneficial effect they need to be effectively delivered alive and/or viable to the small intestine. In general, a composition of the invention therefore does not comprise inactivated bacteria of the species *Blautia,* in particular heat-inactivated bacteria of the species *Blautia.*

The composition of the invention comprises bacteria. In preferred embodiments of the invention, the composition is formulated in freeze-dried form. The composition of the invention may comprise granules or gelatin capsules, for example hard gelatin capsules, comprising a bacterial strain of the invention.

Preferably, the composition of the invention comprises lyophilised bacteria. Lyophilisation of bacteria is a well-established procedure and relevant guidance is available in, for example, references [27-29]. The examples demonstrate that lyophilised compositions are particularly effective.

Alternatively, the composition of the invention may comprise a live, active bacterial culture. The examples demonstrate that cultures of the bacteria of the invention are therapeutically effective.

In some embodiments, the bacterial strain in the composition of the invention has not been inactivated, for example, has not been heat-inactivated. In some embodiments, the bacterial strain in the composition of the invention has not been killed, for example, has not been heat-killed. In some embodiments, the bacterial strain in the composition of the invention has not been attenuated, for example, has not been heat-attenuated. For example, in some embodiments, the bacterial strain in the composition of the invention has not been killed, inactivated and/or attenuated. For example, in some embodiments, the bacterial strain in the composition of the invention is live. For example, in some embodiments, the bacterial strain in the composition of the invention is viable. For example, in some embodiments, the bacterial strain in the composition of the invention is capable of partially or totally colonising the intestine. For example, in some embodiments, the bacterial strain in the composition of the invention is viable and capable of partially or totally colonising the intestine.

The bacterial strain in the composition of the invention is preferably viable. Preferably it is capable of partially or totally colonising the intestine. The bacterial strain in the composition of the invention may be live and viable. The bacterial strain in the composition of the invention may be live, viable and capable of partially or totally colonising the intestine.

In some embodiments, the composition comprises a mixture of live bacterial strains and bacterial strains that have been killed. The invention provides compositions which are formulated to prevent the bacteria from being degraded or absorbed in the upper digestive tract and being unable to exert their effect. For example, the compositions may comprises oxygen scavengers and/or prebiotic substrates, such as vitamin C and non-digestible carbohydrates.

In addition, the composition can be enterically formulated. This ensures that the bacteria are not degraded on the way to the small intestine.

In preferred embodiments, the composition of the invention is encapsulated to enable delivery of the bacterial strain to the intestine. Encapsulation protects the composition from degradation until delivery at the target location through, for example, rupturing with chemical or physical stimuli such as pressure, enzymatic activity, or physical disintegration, which may be triggered by changes in pH. Any appropriate encapsulation method may be used. Exemplary encapsulation techniques include entrapment within a porous matrix, attachment or adsorption on solid carrier surfaces, self-aggregation by flocculation or with cross-linking agents, and mechanical containment behind a microporous membrane or a microcapsule. Guidance on encapsulation that may be useful for preparing compositions of the invention is available in, for example, references [30-31].

The composition may be administered orally and may be in the form of a tablet, capsule or powder. Encapsulated products are preferred because *Blautia* are anaerobes.

A composition of the invention includes a therapeutically effective amount of a bacterial strain of the invention. A therapeutically effective amount of a bacterial strain is sufficient to exert a beneficial effect upon a patient. A therapeutically effective amount of a bacterial strain may be sufficient to result in delivery to and/or partial or total colonisation of the patient's intestine.

A suitable daily dose of the bacteria, for example for an adult human, may be from about 1 × 10³ to about 1 × 10¹¹ colony forming units (CFU); for example, from about 1 × 10⁷ to about 1 × 10¹⁰ CFU; in another example from about 1 × 10⁶ to about 1 × 10¹⁰ CFU; in another example from about 1 × 10⁷ to about 1 × 10¹¹ CFU; in another example from about 1 × 10⁸ to about 1 × 10¹⁰ CFU; in another example from about 1 × 10⁸ to about 1 × 10¹¹ CFU.

In certain embodiments, the dose of the bacteria is at least 10⁹ cells per day, such as at least 10¹⁰, at least 10¹¹, or at least 10¹² cells per day.

A dose of the composition may comprise the bacterial strain from about 1 × 10⁶ to about 1 × 10¹¹ colony forming units (CFU) /g, respect to the weight of the composition. The dose may be suitable for an adult human. For example, the composition may comprise the bacterial strain from about 1 × 10³ to about 1 × 10¹¹ CFU/g; for example, from about 1 × 10⁷ to about 1 × 10¹⁰ CFU/g; in another example from about 1 × 10⁶ to about 1 × 10¹⁰ CFU/g; in another example from about 1 × 10⁷ to about 1 × 10¹¹ CFU/g; in another example from about 1 × 10⁸ to about 1 × 10¹⁰ CFU/g; in another example from about 1 × 10⁸ to about 1 × 10¹¹ CFU/g, from about 1 × 10⁸ to about 1 × 10¹⁰ CFU/g. The dose may be, for example, 1g, 3g, 5g, and 10g. The composition may be formulated as a probiotic. A probiotic is defined by the FAO/WHO as a live microorganism that, when administered in adequate amounts, confers a health benefit on the host.

Typically, a probiotic, such as the composition of the invention, is optionally combined with at least one suitable prebiotic compound. In certain embodiments, the probiotic composition of the present invention includes a prebiotic compound in an amount of from about 1 to about 30% by weight, respect to the total weight composition, (e.g. from 5 to 20% by weight). Known prebiotics include commercial products such as inulin and transgalacto-oligosaccharides.

A prebiotic compound is usually a non-digestible carbohydrate such as an oligo- or polysaccharide, or a sugar alcohol, which is not degraded or absorbed in the upper digestive tract. The carbohydrate may be selected from the group consisting of: fructo-oligosaccharides (or FOS), short-chain fructo-oligosaccharides, inulin, isomalt-oligosaccharides, pectins, xylo-oligosaccharides (or XOS), chitosan-oligosaccharides (or COS), beta-glucans, arable gum modified and resistant starches, polydextrose, D-tagatose, acacia fibers, carob, oats, and citrus fibers. In one aspect, the prebiotics are the short-chain fructo-oligosaccharides (for simplicity shown herein below as FOSs-c.c); said FOSs-c.c. are not digestible carbohydrates, generally obtained by the conversion of the beet sugar and including a saccharose molecule to which three glucose molecules are bonded.

Other prebiotic compounds (such as vitamin C, for example), may be included as oxygen scavengers and to improve the delivery and/or partial or total colonisation and survival *in vivo.* Alternatively, the probiotic composition of the invention may be administered orally as a food or nutritional product, such as milk or whey based fermented dairy product, or as a pharmaceutical product.

The compositions of the invention may comprise pharmaceutically acceptable excipients or carriers. Examples of such suitable excipients may be found in the reference [32]. Acceptable carriers or diluents for therapeutic use are well known in the pharmaceutical art and are described, for example, in reference [33]. Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid, cysteine and esters of p-hydroxybenzoic acid, for example, in some embodiments the preservative is selected from sodium benzoate, sorbic acid and esters of p-hydroxybenzoic acid. Antioxidants and suspending agents may be also used. A further example of a suitable carrier is saccharose. A further example of a preservative is cysteine.

The compositions of the invention may be formulated as a food product. For example, a food product may provide nutritional benefit in addition to the therapeutic effect of the invention, such as in a nutritional supplement. Similarly, a food product may be formulated to enhance the taste of the composition of the invention or to make the composition more attractive to consume by being more similar to a common food item, rather than to a pharmaceutical composition. In certain embodiments, the composition of the invention is formulated as a milk-based product. The term "milk-based product" means any liquid or semi-solid milk- or whey-based product having a varying fat content. The milk-based product can be, e.g., cow's milk, goat's milk, sheep's milk, skimmed milk, whole milk, milk recombined from powdered milk and whey without any processing, or a processed product, such as yoghurt, curdled milk, curd, sour milk, sour whole milk, butter milk and other sour milk products. Another important group includes milk beverages, such as whey beverages, fermented milks, condensed milks, infant or baby milks, flavoured milks, ice cream, milk-containing food such as sweets.

In some embodiments, the compositions of the invention comprise one or more bacterial strains of the species *Blautia hydrogenotrophica* and do not contain bacteria from any other genus or comprise only *de minimis* or biologically irrelevant amounts of bacteria from another genus.

In certain embodiments, the compositions of the invention contain a single bacterial species and do not contain any other bacterial species. In certain embodiments, the compositions of the invention contain a single bacterial strain and do not contain any other bacterial strains. For example, the compositions of the invention may comprise bacteria only of the species *Blautia hydrogenotrophica.* Such compositions may comprise only *de minimis* or biologically irrelevant amounts of other bacterial strains or species. Such compositions may be a culture that is substantially free from other species of organism. In some embodiments, such compositions may be a lyophilisate that is substantially free from other species of organism.

In some embodiments, the composition does not comprise bacteria of the species *Clostridium* and/or does not comprise bacteria of the species *Blautia producta.*

In certain embodiments, the compositions of the invention comprise one or more bacterial strains of the species *Blautia hydrogenotrophica,* and do not contain any other bacterial genus, or which comprise only *de minimis* or biologically irrelevant amounts of bacteria from another genus.

In some embodiments, the compositions of the invention comprise more than one bacterial strain or species. For example, in some embodiments, the compositions of the invention comprise more than one strain from within the same species (e.g. more than 1, 2, 3, 4, 5, 6, 7, 8, 9, 10, 15, 20, 25, 30, 35, 40 or 45 strains), and, optionally, do not contain bacteria from any other species. In some embodiments, the compositions of the invention comprise less than 50 strains from within the same species (e.g. less than 45, 40, 35, 30, 25, 20, 15, 12, 10, 9, 8, 7, 6, 5, 4 or 3 strains), and, optionally, do not contain bacteria from any other species. In some embodiments, the compositions of the invention comprise 1-40, 1-30, 1-20, 1-19, 1-18, 1-15, 1-10, 1-9, 1-8, 1-7, 1-6, 1-5, 1-4, 1-3, 1-2, 2-50, 2-40, 2-30, 2-20, 2-15, 2-10, 2-5, 6-30, 6-15, 16-25, or 31-50 strains from within the same species and, optionally, do not contain bacteria from any other species. In some embodiments, the compositions of the invention comprise more than one species from within the same genus (e.g. more than 1, 2, 3, 4, 5, 6, 7, 8, 9, 10, 12, 15, 17, 20, 23, 25, 30, 35 or 40 species), and, optionally, do not contain bacteria from any other genus. In some embodiments, the compositions of the invention comprise less than 50 species from within the same genus (e.g. less than 50, 45, 40, 35, 30, 25, 20, 15, 12, 10, 8, 7, 6, 5, 4 or 3 species), and, optionally, do not contain bacteria from any other genus. In some embodiments, the compositions of the invention comprise 1-50, 1-40, 1-30, 1-20, 1-15, 1-10, 1-9, 1-8, 1-7, 1-6, 1-5, 1-4, 1-3, 1-2, 2-50, 2-40, 2-30, 2-20, 2-15, 2-10, 2-5, 6-30, 6-15, 16-25, or 31-50 species from within the same genus and, optionally, do not contain bacteria from any other genus. The invention comprises any combination of the foregoing.

In some embodiments, the composition comprises a microbial consortium. For example, in some embodiments, the composition comprises the *Blautia hydrogenotrophica* bacterial strain as part of a microbial consortium. For example, in some embodiments, the *Blautia hydrogenotrophica* bacterial strain is present in combination with one or more (e.g. at least 2, 3, 4, 5, 10, 15 or 20) other bacterial strains from the genus *Blautia* and/or other genera with which it can live symbiotically *in vivo* in the intestine. For example, in some embodiments, the composition comprises a bacterial strain of *Blautia hydrogenotrophica* in combination with a bacterial strain from a different genus. In another example, the composition comprises a bacterial strain of *Blautia hydrogenotrophica* in combination with a bacterial strain from the genus *Blautia* or the composition comprises a bacterial strain of *Blautia hydrogenotrophica* in combination with a bacterial strain from the genus *Blautia* and a bacterial strain from a different genus. In some embodiments, the microbial consortium comprises two or more bacterial strains obtained from a faeces sample of a single organism, e.g. a human. In some embodiments, the microbial consortium is not found together in nature. For example, in some embodiments, the microbial consortium comprises bacterial strains obtained from faeces samples of at least two different organisms. In some embodiments, the two different organisms are from the same species, e.g. two different humans. In some embodiments, the two different organisms are an infant human and an adult human. In some embodiments, the two different organisms are a human and a non-human mammal.

In some embodiments, the composition comprises the *Blautia hydrogenotrophica* bacterial strain as part of a microbial consortium which contains fewer than 5, 10, 11, 12, 13, 14, 15, 16, 17, 18, 19, 20, 30, 40, 50, 60, 70, 80, 90, 100 or 200 bacterial species. The bacterial species may be from the genus *Blautia* and/or from other genera with which it can live symbiotically *in vivo* in the intestine. Those skilled in the art will recognise that a bacterial population comprising 200 or fewer different species is significantly less complex and more controlled than material derived from a faecal sample which will contain many thousands of different species of bacteria. In preferred embodiments, the composition is not a faecal sample, such as a human faecal sample.

In preferred embodiments, the composition may be formulated as part of a microbial consortium which contains fewer than 10, 11, 12, 13, 14, 15, 16, 17, 28, 19, 20, 30, 40 or 50 bacterial strains. The bacterial strains may be from the genus *Blautia* and/or from other genera with which it can live symbiotically *in vivo* in the intestine. In some embodiments, the microbial consortium contains fewer than 30 bacterial strains. In some embodiments, the microbial consortium contains fewer than 20 bacterial strains. In some embodiments, the microbial consortium contains fewer than 10 bacterial strains. In some embodiments, the composition of the invention additionally comprises a bacterial strain that has the same safety and therapeutic efficacy characteristics as the *Blautia hydrogenotrophica* strain deposited under accession number DSM 14294, but which is not the *Blautia hydrogenotrophica* strain deposited under accession number DSM 14294, or which is not a *Blautia hydrogenotrophica* or which is not a *Blautia.*

In some embodiments in which the composition of the invention comprises more than one bacterial strain, species or genus, the individual bacterial strains, species or genera may be for separate, simultaneous or sequential administration. For example, the composition may comprise all of the more than one bacterial strain, species or genera, or the bacterial strains, species or genera may be stored separately and be administered separately, simultaneously or sequentially. In some embodiments, the more than one bacterial strains, species or genera are stored separately but are mixed together prior to use.

In some embodiments, the bacterial strain for use in the invention is obtained from human adult faeces. In some embodiments in which the composition of the invention comprises more than one bacterial strain, all of the bacterial strains are obtained from human adult faeces or if other bacterial strains are present, they are present only in *de minimis* amounts. The bacteria may have been cultured subsequent to being obtained from the human adult faeces and being used in a composition of the invention.

In some embodiments, the one or more *Blautia* bacterial strains is/are the only therapeutically active agent(s) in a composition of the invention. In some embodiments, the bacterial strain(s) in the composition is/are the only therapeutically active agent(s) in a composition of the invention.

The compositions for use in accordance with the invention may or may not require marketing approval.

In certain embodiments, the invention provides the above pharmaceutical composition, wherein said bacterial strain is lyophilised. In certain embodiments, the invention provides the above pharmaceutical composition, wherein said bacterial strain is spray dried. In certain embodiments, the invention provides the above pharmaceutical composition, wherein the bacterial strain is lyophilised or spray dried and wherein it is live. In certain embodiments, the invention provides the above pharmaceutical composition, wherein the bacterial strain is lyophilised or spray dried and wherein it is viable. In certain embodiments, the invention provides the above pharmaceutical composition, wherein the bacterial strain is lyophilised or spray dried and wherein it is capable of partially or totally colonising the intestine. In certain embodiments, the invention provides the above pharmaceutical composition, wherein the bacterial strain is lyophilised or spray dried and wherein it is viable and capable of partially or totally colonising the intestine.

In some cases, the lyophilised or spray dried bacterial strain is reconstituted prior to administration. In some cases, the reconstitution is by use of a diluent described herein.

The compositions of the invention can comprise pharmaceutically acceptable excipients, diluents or carriers.

In certain embodiments, the invention provides a pharmaceutical composition comprising: a bacterial strain as discussed earlier; and a pharmaceutically acceptable excipient, carrier or diluent; wherein the bacterial strain is in an amount sufficient to treat a disorder when administered to a subject in need thereof; and wherein the disorder is sensory hypersensitivity, such as sensory hypersensitivity associated with neuropathy, complex regional pain syndrome, postherpetic neuralgia, fibromyalgia, or migraine. Preferably, the disorder is fibromyalgia. The disorder may be allodynia and/or hyperalgesia, such as allodynia and/or hyperalgesia associated with neuropathy, complex regional pain syndrome, postherpetic neuralgia, fibromyalgia, or migraine. Preferably, the disorder is fibromyalgia.

In certain embodiments, the invention provides the above pharmaceutical composition, wherein the amount of the bacterial strain is from about 1 × 10³ to about 1 × 10¹¹ colony forming units per gram with respect to a weight of the composition.

In certain embodiments, the invention provides the above pharmaceutical composition, wherein the composition is administered at a dose of 1 g, 3 g, 5 g or 10 g.

In certain embodiments, the invention provides the above pharmaceutical composition, wherein the composition is administered by a method selected from the group consisting of oral, rectal, subcutaneous, nasal, buccal, and sublingual.

In certain embodiments, the invention provides the above pharmaceutical composition, comprising a carrier selected from the group consisting of lactose, starch, glucose, methyl cellulose, magnesium stearate, mannitol and sorbitol.

In certain embodiments, the invention provides the above pharmaceutical composition, comprising a diluent selected from the group consisting of ethanol, glycerol and water.

In certain embodiments, the invention provides the above pharmaceutical composition, comprising an excipient selected from the group consisting of starch, gelatin, glucose, anhydrous lactose, free-flow lactose, beta-lactose, corn sweetener, acacia, tragacanth, sodium alginate, carboxymethyl cellulose, polyethylene glycol, sodium oleate, sodium stearate, magnesium stearate, sodium benzoate, sodium acetate and sodium chloride.

In certain embodiments, the invention provides the above pharmaceutical composition, further comprising at least one of a preservative, an antioxidant and a stabilizer.

In certain embodiments, the invention provides the above pharmaceutical composition, comprising a preservative selected from the group consisting of sodium benzoate, sorbic acid and esters of p-hydroxybenzoic acid.

In certain embodiments, the invention provides the above pharmaceutical composition, wherein said bacterial strain is lyophilised.

In certain embodiments, the invention provides the above pharmaceutical composition, wherein when the composition is stored in a sealed container at about 4°C or about 25°C and the container is placed in an atmosphere having 50% relative humidity, at least 80% of the bacterial strain as measured in colony forming units, remains after a period of at least about: 1 month, 3 months, 6 months, 1 year, 1.5 years, 2 years, 2.5 years or 3 years.

In some embodiments, the composition of the invention is provided in a sealed container comprising a composition as described herein. In some embodiments, the sealed container is a sachet or bottle. In some embodiments, the composition of the invention is provided in a syringe comprising a composition as described herein.

The composition of the present invention may, in some embodiments, be provided as a pharmaceutical formulation. For example, the composition may be provided as a tablet or capsule. In some embodiments, the capsule is a gelatine capsule ("gel-cap"). The capsule can be a hard or a soft capsule. In some embodiments, the formulation is a soft capsule. Soft capsules are capsules which may, owing to additions of softeners, such as, for example, glycerol, sorbitol, maltitol and polyethylene glycols, present in the capsule shell, have a certain elasticity and softness. Soft capsules can be produced, for example, on the basis of gelatine or starch. Gelatine-based soft capsules are commercially available from various suppliers. Depending on the method of administration, such as, for example, orally or rectally, soft capsules can have various shapes, they can be, for example, round, oval, oblong or torpedo-shaped. Soft capsules can be produced by conventional processes, such as, for example, by the Scherer process, the Accogel process or the droplet or blowing process.

In some embodiments, the compositions of the invention are administered orally. Oral administration may involve swallowing, so that the compound enters the gastrointestinal tract.

Pharmaceutical formulations suitable for oral administration include solid plugs, solid microparticulates, semi-solid and liquid (including multiple phases or dispersed systems) such as tablets; soft or hard capsules containing multi- or nano-particulates, liquids (e.g. aqueous solutions), emulsions or powders; lozenges (including liquid-filled); chews; gels; fast dispersing dosage forms; films; ovules; sprays; and buccal/mucoadhesive patches.

In some embodiments the pharmaceutical formulation is an enteric formulation, i.e. a gastro-resistant formulation (for example, resistant to gastric pH) that is suitable for delivery of the composition of the invention to the intestine by oral administration. Enteric formulations may be particularly useful when the bacteria or another component of the composition is acid-sensitive, e.g. prone to degradation under gastric conditions.

In some embodiments, the enteric formulation comprises an enteric coating. In some embodiments, the formulation is an enteric-coated dosage form. For example, the formulation may be an enteric-coated tablet or an enteric-coated capsule, or the like. The enteric coating may be a conventional enteric coating, for example, a conventional coating for a tablet, capsule, or the like for oral delivery. The formulation may comprise a film coating, for example, a thin film layer of an enteric polymer, e.g. an acid-insoluble polymer.

In some embodiments, the enteric formulation is intrinsically enteric, for example, gastro-resistant without the need for an enteric coating. Thus, in some embodiments, the formulation is an enteric formulation that does not comprise an enteric coating. In some embodiments, the formulation is a capsule made from a thermogelling material. In some embodiments, the thermogelling material is a cellulosic material, such as methylcellulose, hydroxymethylcellulose or hydroxypropylmethylcellulose (HPMC). In some embodiments, the capsule comprises a shell that does not contain any film forming polymer. In some embodiments, the capsule comprises a shell and the shell comprises hydroxypropylmethylcellulose and does not comprise any film forming polymer (e.g. see [34]). In some embodiments, the formulation is an intrinsically enteric capsule (for example, Vcaps^{®} from Capsugel).

### Culturing methods

The bacterial strains for use in the present invention can be cultured using standard microbiology techniques as detailed in, for example, references [35-37].

The solid or liquid medium used for culture may for example be YCFA agar or YCFA medium. YCFA medium may include (per 100ml, approximate values): Casitone (1.0 g), yeast extract (0.25 g), NaHCO₃ (0.4 g), cysteine (0.1 g), K₂HPO₄ (0.045 g), KH₂PO₄ (0.045 g), NaCl (0.09 g), (NH₄)₂SO₄ (0.09 g), MgSO₄ · 7H₂O (0.009 g), CaCl₂ (0.009 g), resazurin (0.1 mg), hemin (1 mg), biotin (1 µg), cobalamin (1 µg), p-aminobenzoic acid (3 µg), folic acid (5 µg), and pyridoxamine (15 µg).

### General

The practice of the present invention will employ, unless otherwise indicated, conventional methods of chemistry, biochemistry, molecular biology, immunology and pharmacology, within the skill of the art. Such techniques are explained fully in the literature. See, *e.g.,* references [38-45], *etc.*

The term "comprising" encompasses "including" as well as "consisting" e.g. a composition "comprising" X may consist exclusively of X or may include something additional e.g. X + Y.

The term "about" in relation to a numerical value x is optional and means, for example, x+10%.

The word "substantially" does not exclude "completely" e.g. a composition which is "substantially free" from Y may be completely free from Y. Where necessary, the word "substantially" may be omitted from the definition of the invention.

References to a percentage sequence identity between two nucleotide sequences means that, when aligned, that percentage of nucleotides are the same in comparing the two sequences. This alignment and the percent homology or sequence identity can be determined using software programs known in the art, for example those described in section 7.7.18 of ref. [46]. A preferred alignment is determined by the Smith-Waterman homology search algorithm using an affine gap search with a gap open penalty of 5 or 12 (most preferably 12) and a gap extension penalty of 2, BLOSUM matrix of 62. The Smith-Waterman homology search algorithm is disclosed in ref. [47].

Unless specifically stated, a process or method comprising numerous steps may comprise additional steps at the beginning or end of the method, or may comprise additional intervening steps. Also, steps may be combined, omitted or performed in an alternative order, if appropriate.

Various embodiments of the invention are described herein. It will be appreciated that the features specified in each embodiment may be combined with other specified features, to provide further embodiments. In particular, embodiments highlighted herein as being suitable, typical or preferred may be combined with each other (except when they are mutually exclusive).

Any reference to a method for treatment comprising administering an agent to a patient, also covers that agent for use in said method for treatment, as well as the use of the agent in said method for treatment, and the use of the agent in the manufacture of a medicament.

The following examples are offered for illustrative purposes only, and are not intended to limit the scope of the present invention in any way.

### MODES FOR CARRYING OUT THE INVENTION

### Example 1 - Evaluation of two test compound in an acid-saline rat model of fibromyalgia

### Summary

Using the acid-saline rat model of fibromyalgia, the animals received either a composition comprising *Blautia* or one of several controls. The compositions of the invention were found inter alia to reduce mechanical allodynia in this rat model, indicating a reduction in fibromyalgia.

### Animal model

The experiments used the acid-saline rat model of fibromyalgia using male Sprague-Dawley SD rats (n = 12/group). In this model, non-inflammatory pain is induced by repeated injections of acid saline (pH 4.0) separated by 5 days. To this end, rats were anesthetized with isoflurane (2-4%) prior to injection of 100 µL of pH 4.0 saline into the left gastrocnemius muscle. Five days later, the process was repeated; hyperalgesia develops within several hours of the 2^{nd} injection. Model induction was performed for all treatment groups with the exception of Normal Control rats, who underwent a sham injection procedure (pH 7.2 saline) and received an equivalent volume of vehicle (*i.e.* phosphate buffered saline; PBS) delivered in an identical manner to that used for the test article (*i.e.* daily oral gavage).

### Study design

Animals were acclimated to the facility and experimenter handling for a minimum of 7 days, during which time rats received the initial exposure to sucrose water to prevent later neophobic effects and allow for assessment of baseline water consumption. At this time, rats were randomly assigned to treatment groups based on body weight as follows:

| **Group** | **Model Induction** | **Treatment** | **Purpose** | **# of Animals** |
|---|---|---|---|---|
| A | 100 µL saline pH 4.0 | *Blautia hydrogenotrophica* (*p.o*) + Saline (*s.c*) | Experimental | 12 |
| B | 100 µL saline pH 4.0 | Lyobuffer (*p.o*) + Saline (*s.c*) | Experimental | 12 |
| C | 100 µL saline pH 7.2 | PBS (*p.o*) + Saline (*s.c*) | Normal Control | 12 |
| D | 100 µL saline pH 4.0 | PBS (*p.o*) + Saline (*s.c*) | Disease Control | 12 |
| E | 100 µL saline pH 4.0 | PBS (*p.o*) + Buprenorphine in Saline (s.c) | Positive Control | 12 |

On the final day of acclimation, blood and faecal samples were obtained.

Following the acclimation phase, daily oral gavage administration of the appropriate test article or vehicle commenced according to assigned groups (see table above). Daily administration continued throughout the remainder of the study.

During the second treatment week, evaluation of mechanical hyperalgesia (*i.e.* Von Frey monofilament assessment) was performed to provide a baseline measure. During this week, pre-induction sucrose consumption was also measured in preparation for later anhedonia testing. Blood and faecal samples were obtained at the conclusion of the two full weeks of treatment.

Model induction began at the third week of treatment. A cage side pain assessment was performed 2x per day for the first week following model induction, and then 1x per day for the remainder of the study. Mechanical hyperalgesia was evaluated at 24- and 72-hours post-induction, and again at 1 and 2 weeks, while somatosensory hind-paw placements, functional movement assessments, and anhedonia testing were performed weekly. At the conclusion of behavioural testing, a final set of blood and faecal samples was obtained the day before euthanasia.

On the day of euthanasia, tissues were harvested and prepared for subsequent analysis.

### Strain

*Blautia hydrogenotrophica* (BH) strain DSM 14294.

### Weight

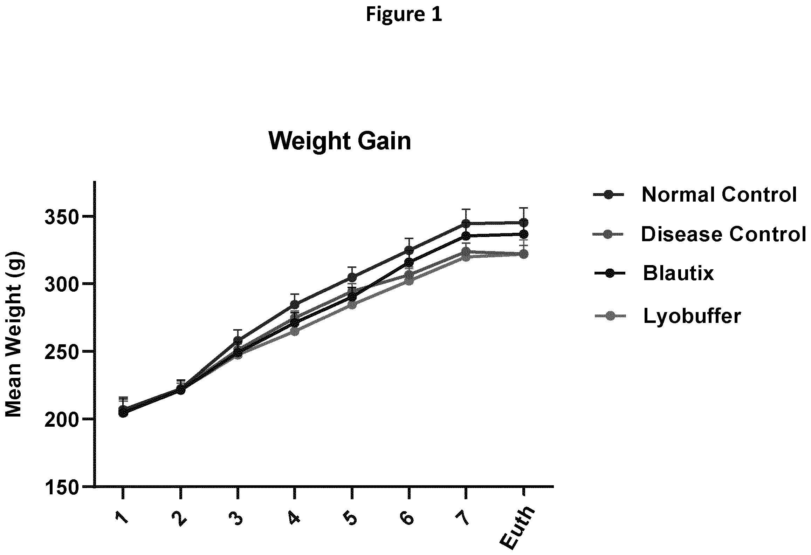

Rats were weighed 24 hours after arrival in the facility, and a minimum of 1× per week thereafter. Figure 1 shows no difference in weight between the groups at the initiation of the study (Normal Control: 206.80 ± 9.10g; Disease Control: 206.70 ± 6.54g; *Blautia hydrogenotrophica:* 204.50 ± 10.33g; Lyobuffer: 206.80 ± 9.31g). While most rats lost some weight at 24-hours post-induction, this loss did not differ between groups and all rats continued to gain weight through the following weeks. By the end of the experiment a trend was observed that the *Blautia hydrogenotrophica* treated and normal control animals gained more weight compared to the disease control and Lyobuffer treated animals.

### Pain

Pain scores were assigned 2x a day for the first post-induction week, and then 1× per day for the duration of the study. Scoring is performed cage side using a 4-point rat grimace scale [48]. Evaluations include the presence/absence of orbital tightening and nose/cheek flattening, as well as positioning of ears and whiskers.

### Sensorimotor Function

Tactile hind-paw placement allows for assessment of sensorimotor integration in response to tactile and proprioceptive stimuli. Scoring was performed once per week following model induction, for a total of 2 sessions. To perform the test, rats are held in the experimenter's hand with limbs hanging freely, and the body held at a 45° angle to a table edge. The animal is brought upwards toward the surface of the table, until the dorsal portion of the hind-paw touches the table. A normal response is to immediately place the paw on the table. The day's score is recorded as a mean of 5 trials.

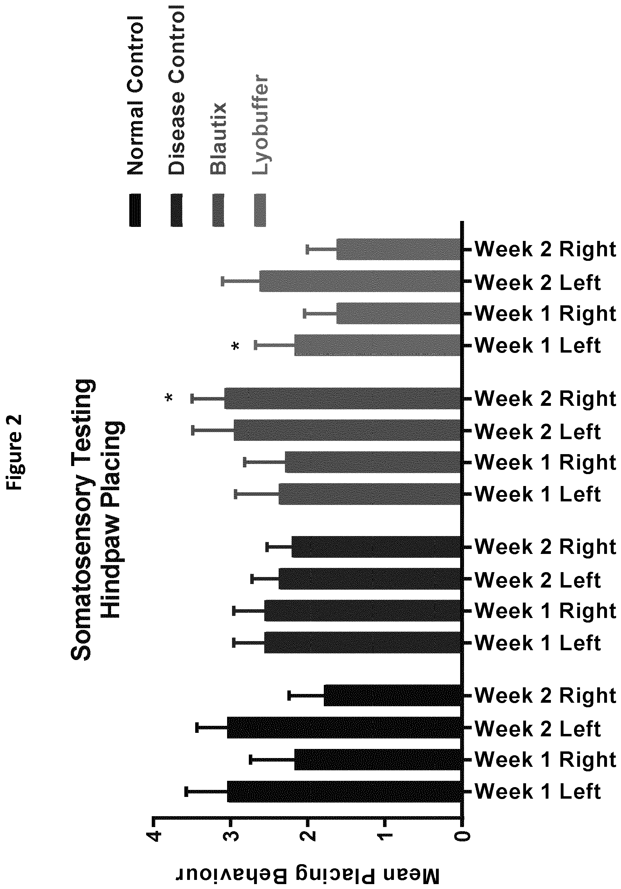

Figure 2 shows that surprisingly *Blautia hydrogenotrophica* treated rats showed improved performance in Week 2, a phenomenon that was not present in any other group. In the right (ipsilateral to injection) paw, this improvement reached statistical significance compared to both Normal Control and Lyobuffer Groups (p = 0.048 and p = 0.017, respectively; T-Test), and trended toward a difference from Disease Control (p = 0.119; T-Test).

Also, interestingly, a difference was found between the ipsilateral vs. contralateral paw placements within groups. This evaluation indicated that Normal Control rats placed less effectively on the side ipsilateral to injection, a difference that trended toward statistical significance by Week 2 (p = 0.054; paired T-Test). A decrease in placing in the ipsilateral paw compared to contralateral was also noted in the Lyobuffer Group, a difference that was statistically significance in both Week 1 and Week 2 (p = 0.020 for each; paired T-Test). However, no within group difference in placing was seen for either Disease Control or the *Blautia hydrogenotrophica* treated rats, although it is important to note that the Disease Control rats appeared to have equivalent difficulty with both ipsilateral and contralateral placing at both time points, while the *Blautia hydrogenotrophica* treated rats showed improved (non-statistically significant) performance for both paws in Week 2.

### Functional Movement

Hind-limb function is scored in an open arena using a modified version of the 21-point Basso, Beatties, Bresnahan (BBB) Locomotor Rating Scale [49], with assessments performed once per week following model induction, for a total of 2 sessions. Evaluation includes paw placement, stability, toe positioning, limping, and weight support (active and stationary).

### Anhedonia (Sucrose Preference)

Prior to model induction and test article administration, rats were acclimated to sucrose sweetened water, and a baseline level for water consumption were established over a 24-hour period. Following this initial exposure, sucrose preference testing was performed the week prior to model induction, and then once per week after the model is induced. For testing, regular water bottles were replaced with two pre-weighed bottles, one containing regular water, and one with a 1% sucrose solution. After 24-hours, bottles were weighed again, and consumption of each was recorded. A decrease in consumption of a rewarding treat (sucrose water) over a 24-hour presentation period is considered indicative of depressed behaviour (anhedonia). No differences between groups was noted for sucrose consumption.

### Mechanical Allodynia - Von Frey Monofilament Testing

Testing for mechanical allodynia was performed using Von Frey monofilaments, and scored via the Dixon Up-Down method to provide a 50% paw withdrawal threshold. Prior to model induction, no statistically significant difference was noted between assigned groups.

Testing was performed at baseline (i.e. prior to model induction), 24 hours post-induction, and again at 72-hours, 1 week, and 2 weeks for a total of 5 sessions. The testing apparatus consists of a small chamber with a wire mesh (5mm square openings) flooring. The apparatus is elevated to allow for easy monofilament application to the plantar surface of each rat paw. Animals are allowed to acclimate to the testing apparatus for 30-minutes prior to each day's test run. Monofilament testing range for rat is 3.61 - 5.18 (0.41 - 15.1g bending force). Testing begins with a 2-second application of the 4.56 (midrange) monofilament, perpendicular to the plantar region of the ipsilateral hind-paw, and with enough pressure to ensure the monofilament fibre bends. Rats are observed during monofilament application, and for 2-seconds following. A positive response is a rapid withdrawal of the hind-paw and may include shaking or licking of the paw. If the response is ambiguous, the same monofilament is reapplied after a 30-second delay. If the response is positive, the next lightest monofilament is assessed, while a negative response is followed by application of the next heavier monofilament. Time between applications is 10-seconds (with the exception of an ambiguous response as outlined above). Six readings are obtained for analysis. Data analysis is via the "up-down" method [50]. Statistically significant differences from Normal Control are indicated by the asterisk.

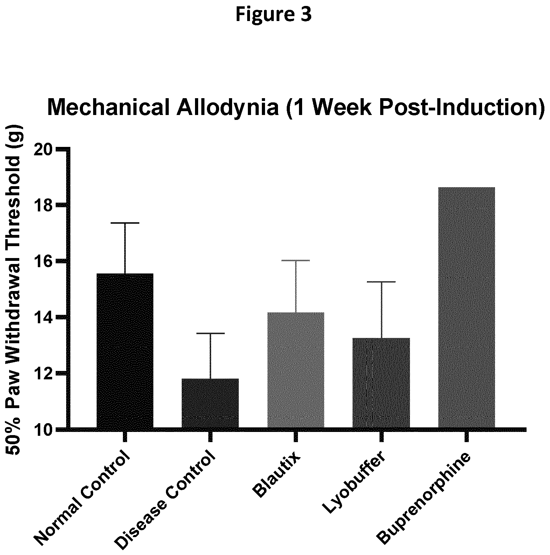

Figure 3 shows the results of the Von Frey monofilament assessment. Administration of compositions comprising *Blautia hydrogenotrophica* led to a notable reduction in mechanical allodynia compared to the control groups.

### Muscle Hyperalgesia

Mechanical nociceptive threshold in the gastrocnemius muscle was quantified at two-weeks post-induction using a digital compression gauge applied to the ipsilateral gastrocnemius muscle until withdrawal of the limb occurred. Three consecutive trials were performed and averaged. The compression force applied at the time of withdrawal was recorded as the compression withdrawal threshold.

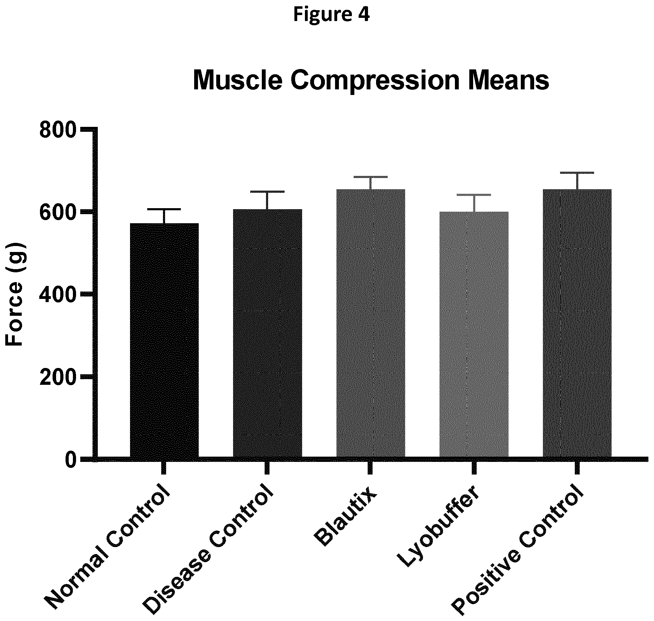

As depicted in Figure 4, there was no main effect for this test at this time point. Overall, rats were less responsive than expected during testing, with compression scores to withdrawal 200 to 400g higher on average than during the Pilot Study. It should be noted that, although there were no between groups differences in this test, the *Blautia hydrogenotrophica* treated rats performed equivalently to Positive Control animals.

### Collection of Blood at Termination

In addition to in-life blood draws described above, a final cardiac blood collection was performed at euthanasia while rats were under anaesthesia. Preparation of plasma samples was identical to that described above (i.e. both EDTA and lithium-heparin plasma), with samples stored at -80°C.

### Collection of Dorsal Root Ganglion at Termination

At the conclusion of the *in vivo* portion of the study, rats were deeply anesthetized with isoflurane, intestinal samples were collected, and dorsal root ganglion (DRG) was dissected out. Following tissue harvest, animals were euthanized via decapitation.

DRG was dissected out, submersion fixed in 10% neutral buffered formalin (NBF), cryoprotected in a 30% w/v sucrose solution, frozen and embedded in OCT media, and stored at -80°C. Three 10µm thick sections per DRG/animal were cut using a cryostat (-21°C), and immunohistochemistry IHC analysis was performed on DRG sections from 8 rats in each treatment group using validated markers for TNF-α and GFAP.

### Collection of Gut Tissue at Termination

Approximately 24 hours after the final administration of test article, rats were deeply anesthetized with isoflurane and the following organs were harvested as quickly as possible:
- The ileum was excised 0.5 cm upstream of the caecum, cut longitudinally, rinsed (flushed with saline solution), and the fat and the contents were removed carefully. Three equal pieces of 1.0 cm each were collected:
   - Proximal sample were placed in RNALater^{™}.
   - Middle sample were snap-frozen in liquid nitrogen.
   - Distal sample were immersion-fixed in 4% paraformaldehyde (PFA), cryoprotected in graded sucrose solution (15-30%) and embedded in OCT and frozen.
- The caecum was excised whole with its contents and snap-frozen in liquid nitrogen.
- The colon was excised 0.5 cm downstream of the caecum, cut longitudinally, rinsed (flushed with saline solution), and the fat and the contents were removed carefully. Three equal pieces of 1.0 cm each of the ascending colon were collected:
   - Proximal sample was placed in RNA Later^{™}.
   - Middle sample was snap-frozen in liquid nitrogen.
   - Distal sample was immersion-fixed in 4% paraformaldehyde (PFA), cryoprotected in graded sucrose solution (15-30%), embedded in OCT and frozen.
- Transverse colon was collected whole, cut longitudinally, rinsed (flushed with saline solution) to remove contents, and snap-frozen in liquid nitrogen.
- Descending colon was collected whole, cut longitudinally, rinsed (flushed with saline solution) to remove contents, and snap-frozen in liquid nitrogen.

### Additional Measures

Two additional measures related to animal welfare were evaluated during this study. Pain was scored cage-side using a 4-point grimace scale 2x per day for the first post-induction week, and then 1× per day for the duration of the study, while functional movement was assessed via a modified Locomotor Rating Scale with assessments performed once per week following model induction for a total of 2 sessions. No deficits were noted in either of these measures, with all animals exhibiting no overt signs of unelicited pain or distress at any time point. **Table 2** provides these data.

**Table 2: Measures of Animal Welfare/Model Parameters**

| **Test** | **Normal Control** | **Disease Control** | ***Blautia hydrogenotrophica*** | **Lyobuffer** |
|---|---|---|---|---|
| **Pain Assessment** | 0 | 0 | 0 | 0 |
| **Functional Movement** | 7 | 7 | 7 | 7 |

### Conclusions

The results from this study support the conclusion that the oral administration of *Blautia hydrogenotrophica* has beneficial effects on several factors conducive to promoting a more positive outcome in the acid-saline model of fibromyalgia. Lyobuffer treatment did not offer any protection, consistently resulting in performance no better than Disease Control levels. It is important to note that, while differences seen in *Blautia hydrogenotrophica* treated animals did not achieve statistical significance compared to Disease Control, this does not preclude their biological significance, especially since this model is by nature inherently variable.

Several results support the conclusion from this study. First, Normal Control and *Blautia hydrogenotrophica* treated rats showed a trend toward an increased weight gain following model induction compared to Disease Control and Lyobuffer Groups.

In behavioural tests, *Blautia hydrogenotrophica* treated rats performed at or better than Normal Control levels, with neither of these Groups exhibiting a statistically significant difference from the Positive Control. Additionally, somatosensory testing indicated that *Blautia hydrogenotrophica* treatment appeared to improve performance by Week 2 post-induction, an achievement not noted in any other group.

The lack of statistical differences between Normal Control and Disease Control Groups, while not anticipated based on earlier pilot work, may be a consequence of the increased handling required throughout this project. Daily administration of experimental compounds via oral gavage before model induction and continuing throughout the remaining weeks of the study, as well as repeated blood draws, required animals to be handled and restrained regularly, often multiple times per day. In addition, the behavioural testing schedule necessitated repeated handling above what occurred during the shorter, more simplified pilot study. Reports in human literature indicate that exercise may be beneficial for modulating symptoms of fibromyalgia [51]. In rats, exercise also has been shown to attenuate pain response [52]. In the current project, measurable changes were often observed when comparing Disease Control to Positive Control animals. Given these results, it is possible that the model simply lacked the robustness necessary in the 2^{nd} post-induction week for detection of more subtle alterations.

### Sequences

SEQ ID NO:1 (*Blautia stercoris* GAM6-1 16S ribosomal RNA gene, partial sequence - HM626177) 
1 tgcaagtcga gcgaagcgct tacgacagaa ccttcggggg aagatgtaag ggactgagcg
61 gcggacgggt gagtaacgcg tgggtaacct gcctcataca gggggataac agttggaaac
121 ggctgctaat accgcataag cgcacggtat cgcatgatac agtgtgaaaa actccggtgg
181 tatgagatgg acccgcgtct gattagctag ttggaggggt aacggcccac caaggcgacg
241 atcagtagcc ggcctgagag ggtgaacggc cacattggga ctgagacacg gcccagactc
301 ctacgggagg cagcagtggg gaatattgca caatggggga aaccctgatg cagcgacgcc
361 gcgtgaagga agaagtatct cggtatgtaa acttctatca gcagggaaga aaatgacggt
421 acctgactaa gaagccccgg ctaactacgt gccagcagcc gcggtaatac gtagggggca
481 agcgttatcc ggatttactg ggtgtaaagg gagcgtagac ggaagagcaa gtctgatgtg
541 aaaggctggg gcttaacccc aggactgcat tggaaactgt ttttcttgag tgccggagag
601 gtaagcggaa ttcctagtgt agcggtgaaa tgcgtagata ttaggaggaa caccagtggc
661 gaaggcggct tactggacgg taactgacgt tgaggctcga aagcgtgggg agcaaacagg
721 attagatacc ctggtagtcc acgccgtaaa cgatgaatac taggtgttgg ggagcaaagc
781 tcttcggtgc cgcagcaaac gcaataagta ttccacctgg ggagtacgtt cgcaagaatg
841 aaactcaaag gaattgacgg ggacccgcac aagcggtgga gcatgtggtt taattcgaag
901 caacgcgaag aaccttacca agtcttgaca tcgatctgac cggttcgtaa tggaaccttt
961 ccttcgggac agagaagaca ggtggtgcat ggttgtcgtc agctcgtgtc gtgagatgtt
1021 gggttaagtc ccgcaacgag cgcaacccct atcctcagta gccagcaggt gaagctgggc
1081 actctgtgga gactgccagg gataacctgg aggaaggcgg ggacgacgtc aaatcatcat
1141 gccccttatg atttgggcta cacacgtgct acaatggcgt aaacaaaggg aagcgagccc
1201 gcgaggggga gcaaatccca aaaataacgt cccagttcgg actgcagtct gcaactcgac
1261 tgcacgaagc tggaatcgct agtaatcgcg aatcagaatg tcgcggtgaa tacgttcccg
1321 ggtcttgtac acaccgcccg tcacaccatg ggagtcagta acgcccgaag tc
SEQ ID NO:2 (*Blautia wexlerae* strain WAL 14507 16S ribosomal RNA gene, partial sequence - EF036467) 
GCCCCCTACGTATTACCGCGGCTGCTGGCACGTAGTTAGCCGGGGCTTCTTAGTCAGGTACCGTCATTTTCTTCCCT
GCTGATAGAAGTTTACATACCGAGATACTTCTTCCTTCACGCGGCGTCGCTGCATCAGGGTTTCCCCCATTGTGCAA
TATTCCCCACTGCTGCCTCCCGTAGGAGTCTGGGCCGTGTCTCAGTCCCAATGTGGCCGTTCACCCTCTCAGGCCGG
CTACTGATCGTCGCCTTGGTGGGCCGTTACCCCTCCAACTAGCTAATCAGACGCGGGTCCATCTCATACCACCGGAG
TTTTTCACACCAGACCATGCGGTCCTGTGCGCTTATGCGGTATTAGCAGTCATTTCTAACTGTTATCCCCCTGTATG
AGGCAGGTTACCCACGCGTTACTCACCCGTCCGCCGCTCAGTCACAAAGACTTCAATCCGAAGAAATCCGTCTTAGT
GCTTCGCTCGACTGCA
SEQ ID NO:8 - *Blautia coccoides* strain 16S rRNA gene sequence -Contig consesus sequence 2 reads assembled using Geneious

GGTCGCTTCGGCAGCTCTTCCTTTCGGTTAGGTCACTGACTTCGGGCGTTACTGACTCCCATGGTGTGACGGGCGGT
GTGTACAAGACCCGGGAACGTATTCACCGCGGCATTCTGATCCGCGATTACTAGCGATTCCAGCTTCGTGCAGTCGA
GTTGCAGACTGCAGTCCGAACTGGGACGTTATTTTTGGGATTCGCTCAACATCGCTGTCTCGCTTCCCTTTGTTTAC
GCCATTGTAGCACGTGTGTAGCCCAAATCATAAGGGGCATGATGATTTGACGTCGTCCCCGCCTTCCTCCGGGTTAT
CCCCGGCAGTCTCCCTAGAGTGCCCACCATCATGTGCTGGCTACTAAGGATAAGGGTTGCGCTCGTTGCGGGACTTA
ACCCAACATCTCACGACACGAGCTGACGACAACCATGCACCACCTGTCTCCTCTGCCCCGAAGGGAAGTCCCCGTTA
CGGGACGGTCAGAGGGATGTCAAGACTTGGTAAGGTTCTTCGCGTTGCTTCGAATTAAACCACATGCTCCACCGCTT
GTGCGGGTCCCCGTCAATTCCTTTGAGTTTCATTCTTGCGAACGTACTCCCCAGGTGGAATACTTATTGCGTTTGCT
GCGGCACCGAATGGCTTTGCCACCCGACACCTAGTATTCATCGTTTACGGCGTGGACTACCAGGGTATCTAATCCTG
TTTGCTCCCCACGCTTTCGAGCCTCAACGTCAGTTACCGTCCAGTAAGCCGCCTTCGCCACTGGTGTTCCTCCTAAT
ATCTACGCATTTCACCGCTACACTAGGAATTCCGCTTACCTCTCCGGCACTCTAGAACAACAGTTTCCAATGCAGTC
CTGGGGTTAAGCCCCAGCCTTTCACATCAGACTTGCTCTTCCGTCTACGCTCCCTTTACACCCAGTAAATCCGGATA
ACGCTTGCCCCCTACGTATTACCGCGGCTGCTGGCACGTAGTTAGCCGGGGCTTCTTAGTCAGGTACCGTCATTTTC
TTCCCTGCTGATAGAAGTTTACATACCGAGATACTTCTTCCTTCACGCGGCGTCGCTGCATCAGGGTTTCCCCCATT
GTGCAATATTCCCCACTGCTGCCTCCCGTAGGAGTCTGGGCCGTGTCTCAGTCCCAATGTGGCCGTTCACCCTCTCA
GGCCGGCTACTGATCGTCGCCTTGGTGGGCCGTTACCCCTCCAACTAGCTAATCAGACGCGGGTCCATCTCATACCA
CCGGAGTTTTTCACACCAGACCATGCGGTCCTGTGCGCTTATGCGGTATTAGCAGTCATTTCTAACTGTTATCCCCC
TGTATGAGGCAGGTTACCCACGCGTTATCACCCGTCCGCCGCTCAGTCACAAAGACTTCAATCCGAAGAAATCCGTC
TTAGCGCTCCGCTCGACTGCATGGTAGC

### REFERENCES

[1] Spor et al. (2011) Nat Rev Microbiol. 9(4):279-90.
[2] Eckburg et al. (2005) Science. 10;308(5728):1635-8.
[3] Tap et al. (2009), Environ Microbiol, 11(10):2574-84.
[4] Macpherson et al. (2001) Microbes Infect. 3(12):1021-35
[5] Macpherson et al. (2002) Cell Mol Life Sci. 59(12):2088-96.
[6] Mazmanian et al. (2005) Cell 15;122(1):107-18.
[7] Frank et al. (2007) PNAS 104(34): 13780-5.
[8] Scanlan et al. (2006) J Clin Microbiol. 44(11):3980-8.
[9] Kang et al. (2010) Inflamm Bowel Dis. 16(12):2034-42.
[10] Machiels et al. (2013) Gut. 63(8): 1275-83.
[11] Lopetuso et al. (2013), Gut Pathogens, 5: 23
[12] WO 2013/050792
[13] WO 03/046580
[14] WO 2013/008039
[15] WO 2014/167338
[16] Lee and Lee (2014) World J Gastroenterol. 20(27): 8886-8897.
[17] WO 01/85187
[18] Liu et al. (2008) Int J Syst Evol Microbiol 58, 1896-1902.
[19] Bernalier et al. (1996) Arch. Microbiol. 166 (3), 176-183.
[20] Park et al. (2012) Int J Syst Evol Microbiol. 62(Pt 4):776-9.
[21] Masco et al. (2003) Systematic and Applied Microbiology, 26:557-563.
[22] Srûtkova *et al.* (2011) *J. Microbiol. Methods,* 87(1):10-6.
[23] Stanos et al. (2016) Postgraduate Medicine, 128, 5, 502-515
[24] Coyne et al (2017) Postgraduate Medicine, 129, 1, 22-31
[25] Dixon et al. (2016) JBehαv Med, 39:537-550
[26] Pace et al. (2001) Visceral Hypersensitivity Is Not a Feature of Fibromyalgia Syndrome, Journal of Musculoskeletal Pain, 9:1, 47-55.
[27] Miyamoto-Shinohara et al. (2008) J. Gen. Appl. Microbiol., 54, 9-24.
[28] Cryopreservation and Freeze-Drying Protocols, ed. by Day and McLellan, Humana Press.
[29] Leslie et al. (1995) Appl. Environ. Microbiol. 61, 3592-3597.
[30] Mitropoulou et al. (2013) JNutr Metab. (2013) 716861.
[31] Kailasapathy et al. (2002) Curr Issues Intest Microbiol. 3(2):39-48.
[32] Handbook of Pharmaceutical Excipients, 2nd Edition, (1994), Edited by A Wade and PJ Weller
[33] Remington's Pharmaceutical Sciences, Mack Publishing Co. (A. R. Gennaro edit. 1985)
[34] US 2016/0067188
[35] Handbook of Microbiological Media, Fourth Edition (2010) Ronald Atlas, CRC Press.
[36] Maintaining Cultures for Biotechnology and Industry (1996) Jennie C. Hunter-Cevera, Academic Press
[37] Strobel (2009) Methods Mol Biol. 581:247-61.
[38] Gennaro (2000) Remington: The Science and Practice of Pharmacy. 20th edition, ISBN: 0683306472.
[39] Molecular Biology Techniques: An Intensive Laboratory Course, (Ream et al., eds., 1998, Academic Press).
*[40]* Methods In Enzymology (S. Colowick and N. Kaplan, eds., Academic Press, Inc.)
*[41]* Handbook of Experimental Immunology, Vols. I-IV (D.M. Weir and C.C. Blackwell, eds, 1986, Blackwell Scientific Publications)
[42] Sambrook et al. (2001) Molecular Cloning: A Laboratory Manual, 3rd edition (Cold Spring Harbor Laboratory Press).
*[43]* Handbook of Surface and Colloidal Chemistry (Birdi, K.S. ed., CRC Press, 1997)
[44] Ausubel et al. (eds) (2002) Short protocols in molecular biology, 5th edition (Current Protocols).
*[45]* PCR (Introduction to Biotechniques Series), 2nd ed. (Newton & Graham eds., 1997, Springer Verlag)
*[46]* Current Protocols in Molecular Biology (F.M. Ausubel et al., eds., 1987) Supplement 30
[47] Smith & Waterman (1981) Adv. Appl. Math. 2: 482-489.
[48] Sotocinal et al. (2011). Mol Pain, 7: 55. doi: 10.1186/1744-8069-7-55.
[49] Basso et al. (1995). J Neurotrauma, 12:1-21.
[50] Chaplan et al. (1994). JNeurosci Methods, 53; 55-63.
[51] O'Dwyer et αl.(2019) Rheumatology International, https://doi.org/10.1007/s00296-019-04270-3
[52] Allen et al (20017) Arthritis & Rheumatology, 69(7): 1407-1417

### Sequences

SEQ ID NO:1 *(Blautia stercoris* strain GAM6-1 16S ribosomal RNA gene, partial sequence - HM626177) 
1 tgcaagtcga gcgaagcgct tacgacagaa ccttcggggg aagatgtaag ggactgagcg
61 gcggacgggt gagtaacgcg tgggtaacct gcctcataca gggggataac agttggaaac
121 ggctgctaat accgcataag cgcacggtat cgcatgatac agtgtgaaaa actccggtgg
181 tatgagatgg acccgcgtct gattagctag ttggaggggt aacggcccac caaggcgacg
241 atcagtagcc ggcctgagag ggtgaacggc cacattggga ctgagacacg gcccagactc
301 ctacgggagg cagcagtggg gaatattgca caatggggga aaccctgatg cagcgacgcc
361 gcgtgaagga agaagtatct cggtatgtaa acttctatca gcagggaaga aaatgacggt
421 acctgactaa gaagccccgg ctaactacgt gccagcagcc gcggtaatac gtagggggca
481 agcgttatcc ggatttactg ggtgtaaagg gagcgtagac ggaagagcaa gtctgatgtg
541 aaaggctggg gcttaacccc aggactgcat tggaaactgt ttttcttgag tgccggagag
601 gtaagcggaa ttcctagtgt agcggtgaaa tgcgtagata ttaggaggaa caccagtggc
661 gaaggcggct tactggacgg taactgacgt tgaggctcga aagcgtgggg agcaaacagg
721 attagatacc ctggtagtcc acgccgtaaa cgatgaatac taggtgttgg ggagcaaagc
781 tcttcggtgc cgcagcaaac gcaataagta ttccacctgg ggagtacgtt cgcaagaatg
841 aaactcaaag gaattgacgg ggacccgcac aagcggtgga gcatgtggtt taattcgaag
901 caacgcgaag aaccttacca agtcttgaca tcgatctgac cggttcgtaa tggaaccttt
961 ccttcgggac agagaagaca ggtggtgcat ggttgtcgtc agctcgtgtc gtgagatgtt
1021 gggttaagtc ccgcaacgag cgcaacccct atcctcagta gccagcaggt gaagctgggc
1081 actctgtgga gactgccagg gataacctgg aggaaggcgg ggacgacgtc aaatcatcat
1141 gccccttatg atttgggcta cacacgtgct acaatggcgt aaacaaaggg aagcgagccc
1201 gcgaggggga gcaaatccca aaaataacgt cccagttcgg actgcagtct gcaactcgac
1261 tgcacgaagc tggaatcgct agtaatcgcg aatcagaatg tcgcggtgaa tacgttcccg
1321 ggtcttgtac acaccgcccg tcacaccatg ggagtcagta acgcccgaag tc
SEQ ID NO:2 (*Blautia wexlerae* strain WAL 14507 16S ribosomal RNA gene, partial sequence - EF036467) 
1 caagtcgaac gggaattant ttattgaaac ttcggtcgat ttaatttaat tctagtggcg
61 gacgggtgag taacgcgtgg gtaacctgcc ttatacaggg ggataacagt cagaaatggc
121 tgctaatacc gcataagcgc acagagctgc atggctcagt gtgaaaaact ccggtggtat
181 aagatggacc cgcgttggat tagcttgttg gtggggtaac ggcccaccaa ggcgacgatc
241 catagccggc ctgagagggt gaacggccac attgggactg agacacggcc cagactccta
301 cgggaggcag cagtggggaa tattgcacaa tgggggaaac cctgatgcag cgacgccgcg
361 tgaaggaaga agtatctcgg tatgtaaact tctatcagca gggaagatag tgacggtacc
421 tgactaagaa gccccggcta actacgtgcc agcagccgcg gtaatacgta gggggcaagc
481 gttatccgga tttactgggt gtaaagggag cgtagacggt gtggcaagtc tgatgtgaaa
541 ggcatgggct caacctgtgg actgcattgg aaactgtcat acttgagtgc cggaggggta
601 agcggaattc ctagtgtagc ggtgaaatgc gtagatatta ggaggaacac cagtggcgaa
661 ggcggcttac tggacggtaa ctgacgttga ggctcgaaag cgtggggagc aaacaggatt
721 agataccctg gtagtccacg ccgtaaacga tgaataacta ggtgtcgggt ggcaaagcca
781 ttcggtgccg tcgcaaacgc agtaagtatt ccacctgggg agtacgttcg caagaatgaa
841 actcaaagga attgacgggg acccgcacaa gcggtggagc atgtggttta attcgaagca
901 acgcgaagaa ccttaccaag tcttgacatc cgcctgaccg atccttaacc ggatctttcc
961 ttcgggacag gcgagacagg tggtgcatgg ttgtcgtcag ctcgtgtcgt gagatgttgg
1021 gttaagtccc gcaacgagcg caacccctat cctcagtagc cagcatttaa ggtgggcact
1081 ctggggagac tgccagggat aacctggagg aaggcgggga tgacgtcaaa tcatcatgcc
1141 ccttatgatt tgggctacac acgtgctaca atggcgtaaa caaagggaag cgagattgtg
1201 agatggagca aatcccaaaa ataacgtccc agttcggact gtagtctgca acccgactac
1261 acgaagctgg aatcgctagt aatcgcggat cagaatgccg cggtgaatac gttcccgggt
1321 cttgtacaca ccgcccgtca caccatggga gtcagtaacg cccgaagtca gtgacctaac
1381 tgcaaagaag gagctgccga aggcgggacc gatgactggg gtgaagtcgt aacaaggt
SEQ ID NO:3 (consensus 16S rRNA sequence for *Blautia stercoris* strain 830) 
TTTKGTCTGGCTCAGGATGAACGCTGGCGGCGTGCTTAACACATGCAAGTCGAGCGAAGCGCTTACGACAGAACCTT
CGGGGGAAGATGTAAGGGACTGAGCGGCGGACGGGTGAGTAACGCGTGGGTAACCTGCCTCATACAGGGGGATAACA
GTTGGAAACGGCTGCTAATACCGCATAAGCGCACAGTATCGCATGATACAGTGTGAAAAACTCCGGTGGTATGAGAT
GGACCCGCGTCTGATTAGCTAGTTGGAGGGGTAACGGCCCACCAAGGCGACGATCAGTAGCCGGCCTGAGAGGGTGA
ACGGCCACATTGGGACTGAGACACGGCCCAGACTCCTACGGGAGGCAGCAGTGGGGAATATTGCACAATGGGGGAAA
CCCTGATGCAGCGACGCCGCGTGAAGGAAGAAGTATCTCGGTATGTAAACTTCTATCAGCAGGGAAGAAAATGACGG
TACCTGACTAAGAAGCCCCGGCTAACTACGTGCCAGCAGCCGCGGTAATACGTAGGGGGCAAGCGTTATCCGGATTT
ACTGGGTGTAAAGGGAGCGTAGACGGAAGAGCAAGTCTGATGTGAAAGGCTGGGGCTTAACCCCAGGACTGCATTGG
AAACTGTTTTTCTTGAGTGCCGGAGAGGTAAGCGGAATTCCTAGTGTAGCGGTGAAATGCGTAGATATTAGGAGGAA
CACCAGTGGCGAAGGCGGCTTACTGGACGGTAACTGACGTTGAGGCTCGAAAGCGTGGGGAGCAAACAGGATTAGAT
ACCCTGGTAGTCCACGCCGTAAACGATGAATACTAGGTGTTGGGGAGCAAAGCTCTTCGGTGCCGCAGCAAACGCAA
TAAGTATTCCACCTGGGGAGTACGTTCGCAAGAATGAAACTCAAAGGAATTGACGGGGACCCGCACAAGCGGTGGAG
CATGTGGTTTATTCGAAGCAACGCGAAGAACCTTACCAAGTCTTGACATCGATCTGACCGGTTCGTAATGGAACCTT
TCCTTCGGGACAGAGAAGACAGGTGGTGCATGGTTGTCGTCAGCTCGTGTCGTGAGATGTTGGGTTAAGTCCCGCAA
CGAGCGCAACCCCTATCGTCAGTAGCCAGCAGGTAAAGCTGGGCACTCTGAGGAGACTGCCAGGGATAACCTGGAGG
AAGGCGGGGACGACGTCAAATCATCATGCCCCTTATGATTTGGGCTACACACGTGCTACAATGGCGTAAACAAAGGG
AAGCGAGCCCGCGAGGGGGAGCAAATCCCAAAAATAACGTCCCAGTTCGGACTGCAGTCTGCAACTCGACTGCACGA
AGCTGGAATCGCTAGTAATCGCGAATCAGAATGTCGCGGTGAATACGTTCCCGGGTCTTGTACACACCGCCCGTCAC
 
ACCATGGGAGTCAGTAACGCCCGAAGTCAGTGACCCAACCTTAGGGAGGGAGCTGCCGAAGGCGGGATTGATAACTG
GGGTGAAGTCTAGGGGGT
SEQ ID NO:4 (consensus 16S rRNA sequence for *Blautia wexlerae* strain NCIMB 42486) 
TTCATTGAGACTTCGGTGGATTTAGATTCTATTTCTAGTGGCGGACGGGTGAGTAACGCGTGGGTAACCTGCCTTAT
ACAGGGGGATAACAGTCAGAAATGGCTGCTAATACCGCATAAGCGCACAGAGCTGCATGGCTCAGTGTGAAAAACTC
CGGTGGTATAAGATGGACCCGCGTTGGATTAGCTTGTTGGTGGGGTAACGGCCCACCAAGGCGACGATCCATAGCCG
GCCTGAGAGGGTGAACGGCCACATTGGGACTGAGACACGGCCCAGACTCCTACGGGAGGCAGCAGTGGGGAATATTG
CACAATGGGGGAAACCCTGATGCAGCGACGCCGCGTGAAGGAAGAAGTATCTCGGTATGTAAACTTCTATCAGCAGG
GAAGATAGTGACGGTACCTGACTAAGAAGCCCCGGCTAACTACGTGCCAGCAGCCGCGGTAATACGTAGGGGGCAAG
CGTTATCCGGATTTACTGGGTGTAAAGGGAGCGTAGACGGTGTGGCAAGTCTGATGTGAAAGGCATGGGCTCAACCT
GTGGACTGCATTGGAAACTGTCATACTTGAGTGCCGGAGGGGTAAGCGGAATTCCTAGTGTAGCGGTGAAATGCGTA
GATATTAGGAGGAACACCAGTGGCGAAGGCGGCTTACTGGACGGTAACTGACGTTGAGGCTCGAAAGCGTGGGGAGC
AAACAGGATTAGATACCCTGGTAGTCCACGCCGTAAACGATGAATACTAGGTGTCNGGGGAGCATGGCTCTTCGGTG
CCGTCGCAAACGCAGTAAGTATTCCACCTGGGGAGTACGTTCGCAAGAATGAAACTCAAAGGAATTGACGGGGACCC
GCACAAGCGGTGGAGCATGTGGTTTAATTCGAAGCAACGCGAAGAACCTTACCAAGTCTTGACATCCGCCTGACCGA
TCCTTAACCGGATCTTTCCTTCGGGACAGGCGAGACAGGTGGTGCATGGTTGTCGTCAGCTCGTGTCGTGAGATGTT
GGGTTAAGTCCCGCAACGAGCGCAACCCCTATCCTCAGTAGCCAGCATTTAAGGTGGGCACTCTGGGGAGACTGCCA
GGGATAACCTGGAGGAAGGCGGGGATGACGTCAAATCATCATGCCCCTTATGATTTGGGCTACACACGTGCTACAAT
GGCGTAAACAAAGGGAAGCGAGATCGTGAGATGGAGCAAATCCCAAAAATAACGTCCCAGTTCGGACTGTAGTCTGC
AACCCGACTACACGAAGCTGGAATCGCTAGTAATCGCGGATCAGAATGCCGCGGTGAATACGTTCCCGGGTCTTGTA
CACACCGCCCGTCACACCATGGGAGTCAGTAACGCCCGAAGTCAGTGACCTAACTGCAAAGAAGGAGCTGCCGAA
SEQ ID NO:5 (*Blautia hydrogenotrophica* strain S5a36 16S ribosomal RNA gene, partial sequence - X95624.1) 
1 gatgaacgct ggcggcgtgc ttaacacatg caagtcgaac gaagcgatag agaacggaga
61 tttcggttga agttttctat tgactgagtg gcggacgggt gagtaacgcg tgggtaacct
121 gccctataca gggggataac agttagaaat gactgctaat accgcataag cgcacagctt
181 cgcatgaagc ggtgtgaaaa actgaggtgg tataggatgg acccgcgttg gattagctag
241 ttggtgaggt aacggcccac caaggcgacg atccatagcc ggcctgagag ggtgaacggc
301 cacattggga ctgagacacg gcccaaactc ctacgggagg cagcagtggg gaatattgca
361 caatggggga aaccctgatg cagcgacgcc gcgtgaagga agaagtatct cggtatgtaa
421 acttctatca gcagggaaga aagtgacggt acctgactaa gaagccccgg ctaattacgt
481 gccagcagcc gcggtaatac gtaaggggca agcgttatcc ggatttactg ggtgtaaagg
541 gagcgtagac ggtttggcaa gtctgatgtg aaaggcatgg gctcaacctg tggactgcat
601 tggaaactgt cagacttgag tgccggagag gcaagcggaa ttcctagtgt agcggtgaaa
661 tgcgtagata ttaggaggaa caccagtggc gaaggcggcc tgctggacgg taactgacgt
721 tgaggctcga aagcgtgggg agcaaacagg attagatacc ctggtagtcc acgctgtaaa
781 cgatgaatac taggtgtcgg gtggcaaagc cattcggtgc cgcagcaaac gcaataagta
841 ttcccacctg gggagtacgt tcgcaagaat gaaactcaaa ggaattgacg gggacccgca
901 caagcggtgg agcatgtggt ttaattcgaa gcaacgcgaa gaaccttacc aaatcttgac
961 atccctctga ccgggaagta atgttccctt ttcttcggaa cagaggagac aggtggtgca
 
1021 tggttgtcgt cagctcgtgt cgtgagatgt tgggttaagt cccgcaacga gcgcaaccct
1081 tattcttagt agccagcagg tagagctggg cactctaggg agactgccag ggataacctg
1141 gaggaaggtg gggatgacgt caaatcatca tgccccttat gatttgggct acacacgtgc
1201 tacaatggcg taaacaaagg gaagcgaagg ggtgacctgg agcaaatctc aaaaataacg
1261 tctcagttcg gattgtagtc tgcaactcga ctacatgaag ctggaatcgc tagtaatcgc
1321 gaatcagaat gtcgcggtga atacgttccc gggtcttgta cacaccgccc gtcacaccat
1381 gggagtcagt aacgcccgaa gtcagtgacc caaccnaaag gagggagctg ccgaaggtgg
1441 gactgataac tggggtga
SEQ ID NO:6 (*Blautia producta* strain NCΠVIB 43170 16S rRNA gene sequence - consensus) 
GACTTCGGGCGTTACTGACTCCCATGGTGTGACGGGCGGTGTGTACAAGACCCGGGAACGTATTCACCGCGGCATTC
TGATCCGCGATTACTAGCGATTCCAGCTTCGTGCAGTCGAGTTGCAGACTGCAGTCCGAACTGGGACGTTATTTTTG
GGATTCGCTCAACATCGCTGTCTCGCTTCCCTTTGTTTACGCCATTGTAGCACGTGTGTAGCCCAAATCATAAGGGG
CATGATGATTTGACGTCGTCCCCGCCTTCCTCCGGGTTATCCCCGGCAGTCTCCCTAGAGTGCCCACCATCATGTGC
TGGCTACTAAGGATAAGGGTTGCGCTCGTTGCGGGACTTAACCCAACATCTCACGACACGAGCTGACGACAACCATG
CACCACCTGTCTCCTCTGCCCCGAAGGGAAGTCCCCGTTACGGGACGGTCAGAGGGATGTCAAGACTTGGTAAGGTT
CTTCGCGTTGCTTCGAATTAAACCACATGCTCCACCGCTTGTGCGGGTCCCCGTCAATTCCTTTGAGTTTCATTCTT
GCGAACGTACTCCCCAGGTGGAATACTTATTGCGTTTGCTGCGGCACCGAATGGCTTTGCCACCCGACACCTAGTAT
TCATCGTTTACGGCGTGGACTACCAGGGTATCTAATCCTGTTTGCTCCCCACGCTTTCGAGCCTCAACGTCAGTTAC
CGTCCAGTAAGCCGCCTTCGCCACTGGTGTTCCTCCTAATATCTACGCATTTCACCGCTACACTAGGAATTCCGCTT
ACCTCTCCGGCACTCTAGAACAACAGTTTCCAATGCAGTCCTGGGGTTAAGCCCCAGCCTTTCACATCAGACTTGCT
CTTCCGTCTACGCTCCCTTTACACCCAGTAAATCCGGATAACGCTTGCCCCCTACGTATTACCGCGGCTGCTGGCAC
GTAGTTAGCCGGGGCTTCTTAGTCAGGTACCGTCATTTTCTTCCCTGCTGATAGAAGTTTACATACCGAGATACTTC
TTCCTTCACGCGGCGTCGCTGCATCAGGGTTTCCCCCATTGTGCAATATTCCCCACTGCTGCCTCCCGTAGGAGTCT
GGGCCGTGTCTCAGTCCCAATGTGGCCGTTCACCCTCTCAGGCCGGCTACTGATCGTCGCCTTGGTGGGCCGTTACC
CCTCCAACTAGCTAATCAGACGCGGGTCCATCTCATACCACCGGAGTTTTTCACACCAGACCATGCGGTCCTGTGCG
CTTATGCGGTATTAGCAGTCATTTCTAACTGTTATCCCCCTGTATGAGGCAGGTTACCCACGCGTTACTCACCCGTC
CGCCGCTCAGTCACAAAGACTTCAATCCGAAGAAATCCTGTCTTAGTGCTTCGCT
SEQ ID NO:7 - *Blautia coccoides* strain 16S rRNA gene sequence -Contig 15 consensus sequence 2 reads assembled using Geneious 
TCGGCAGCTCCTTCCTTTCGGTTAGGTCACTGACTTCGGGCGTTACTGACTCCCATGGTGTGACGGGCGGTGTGTAC
AAGACCCGGGAACGTATTCACCGCGGCATTCTGATCCGCGATTACTAGCGATTCCAGCTTCGTGCAGTCGAGTTGCA
GACTGCAGTCCGAACTGGGACGTTATTTTTGGGATTCGCTCAACATCGCTGTCTCGCTTCCCTTTGTTTACGCCATT
GTAGCACGTGTGTAGCCCAAATCATAAGGGGCATGATGATTTGACGTCGTCCCCGCCTTCCTCCGGGTTATCCCCGG
CAGTCTCCCTAGAGTGCCCACCATCATGTGCTGGCTACTAAGGATAAGGGTTGCGCTCGTTGCGGGACTTAACCCAA
CATCTCACGACACGAGCTGACGACAACCATGCACCACCTGTCTCCTCTGCCCCGAAGGGAAGTCCCCGTTACGGGAC
GGTCAGAGGGATGTCAAGACTTGGTAAGGTTCTTCGCGTTGCTTCGAATTAAACCACATGCTCCACCGCTTGTGCGG
GTCCCCGTCAATTCCTTTGAGTTTCATTCTTGCGAACGTACTCCCCAGGTGGAATACTTATTGCGTTTGCTGCGGCA
CCGAATGGCTTTGCCACCCGACACCTAGTATTCATCGTTTACGGCGTGGACTACCAGGGTATCTAATCCTGTTTGCT
CCCCACGCTTTCGAGCCTCAACGTCAGTTACCGTCCAGTAAGCCGCCTTCGCCACTGGTGTTCCTCCTAATATCTAC
GCATTTCACCGCTACACTAGGAATTCCGCTTACCTCTCCGGCACTCTAGAACAACAGTTTCCAATGCAGTCCTGGGG
TTAAGCCCCAGCCTTTCACATCAGACTTGCTCTTCCGTCTACGCTCCCTTTACACCCAGTAAATCCGGATAACGCTT
 
GCCCCCTACGTATTACCGCGGCTGCTGGCACGTAGTTAGCCGGGGCTTCTTAGTCAGGTACCGTCATTTTCTTCCCT
GCTGATAGAAGTTTACATACCGAGATACTTCTTCCTTCACGCGGCGTCGCTGCATCAGGGTTTCCCCCATTGTGCAA
TATTCCCCACTGCTGCCTCCCGTAGGAGTCTGGGCCGTGTCTCAGTCCCAATGTGGCCGTTCACCCTCTCAGGCCGG
CTACTGATCGTCGCCTTGGTGGGCCGTTACCCCTCCAACTAGCTAATCAGACGCGGGTCCATCTCATACCACCGGAG
TTTTTCACACCAGACCATGCGGTCCTGTGCGCTTATGCGGTATTAGCAGTCATTTCTAACTGTTATCCCCCTGTATG
AGGCAGGTTACCCACGCGTTACTCACCCGTCCGCCGCTCAGTCACAAAGACTTCAATCCGAAGAAATCCGTCTTAGT
GCTTCGCTCGACTGCA
SEQ ID NO:8 - *Blautia coccoides* strain 16S rRNA gene sequence -Contig consensus sequence 2 reads assembled using Geneious

GGTCGCTTCGGCAGCTCTTCCTTTCGGTTAGGTCACTGACTTCGGGCGTTACTGACTCCCATGGTGTGACGGGCGGT
GTGTACAAGACCCGGGAACGTATTCACCGCGGCATTCTGATCCGCGATTACTAGCGATTCCAGCTTCGTGCAGTCGA
GTTGCAGACTGCAGTCCGAACTGGGACGTTATTTTTGGGATTCGCTCAACATCGCTGTCTCGCTTCCCTTTGTTTAC
GCCATTGTAGCACGTGTGTAGCCCAAATCATAAGGGGCATGATGATTTGACGTCGTCCCCGCCTTCCTCCGGGTTAT
CCCCGGCAGTCTCCCTAGAGTGCCCACCATCATGTGCTGGCTACTAAGGATAAGGGTTGCGCTCGTTGCGGGACTTA
ACCCAACATCTCACGACACGAGCTGACGACAACCATGCACCACCTGTCTCCTCTGCCCCGAAGGGAAGTCCCCGTTA
CGGGACGGTCAGAGGGATGTCAAGACTTGGTAAGGTTCTTCGCGTTGCTTCGAATTAAACCACATGCTCCACCGCTT
GTGCGGGTCCCCGTCAATTCCTTTGAGTTTCATTCTTGCGAACGTACTCCCCAGGTGGAATACTTATTGCGTTTGCT
GCGGCACCGAATGGCTTTGCCACCCGACACCTAGTATTCATCGTTTACGGCGTGGACTACCAGGGTATCTAATCCTG
TTTGCTCCCCACGCTTTCGAGCCTCAACGTCAGTTACCGTCCAGTAAGCCGCCTTCGCCACTGGTGTTCCTCCTAAT
ATCTACGCATTTCACCGCTACACTAGGAATTCCGCTTACCTCTCCGGCACTCTAGAACAACAGTTTCCAATGCAGTC
CTGGGGTTAAGCCCCAGCCTTTCACATCAGACTTGCTCTTCCGTCTACGCTCCCTTTACACCCAGTAAATCCGGATA
ACGCTTGCCCCCTACGTATTACCGCGGCTGCTGGCACGTAGTTAGCCGGGGCTTCTTAGTCAGGTACCGTCATTTTC
TTCCCTGCTGATAGAAGTTTACATACCGAGATACTTCTTCCTTCACGCGGCGTCGCTGCATCAGGGTTTCCCCCATT
GTGCAATATTCCCCACTGCTGCCTCCCGTAGGAGTCTGGGCCGTGTCTCAGTCCCAATGTGGCCGTTCACCCTCTCA
GGCCGGCTACTGATCGTCGCCTTGGTGGGCCGTTACCCCTCCAACTAGCTAATCAGACGCGGGTCCATCTCATACCA
CCGGAGTTTTTCACACCAGACCATGCGGTCCTGTGCGCTTATGCGGTATTAGCAGTCATTTCTAACTGTTATCCCCC
TGTATGAGGCAGGTTACCCACGCGTTATCACCCGTCCGCCGCTCAGTCACAAAGACTTCAATCCGAAGAAATCCGTC
TTAGCGCTCCGCTCGACTGCATGGTAGC

## Claims

1. A composition comprising a bacterial strain of the species *Blautia hydrogenotrophica* for use in:
(a) a method of treating or preventing sensory hypersensitivity in a subject diagnosed with fibromyalgia; or
(b) a method of treating or preventing allodynia and/or hyperalgesia which are both associated with sensory hypersensitivity in a subject diagnosed with fibromyalgia; or
(c) a method of treating or preventing fibromyalgia.

2. The composition for use according to claim 1, wherein the composition is for use in a patient who has not been diagnosed with visceral hypersensitivity.

3. The composition for use according to any preceding claim, wherein the composition is for oral administration.

4. The composition for use according to any preceding claim, wherein the composition comprises one or more pharmaceutically acceptable excipients or carriers.

5. The composition for use according to any preceding claim, wherein the bacterial strain is lyophilised or viable.

6. The composition for use according to any preceding claim, wherein the composition comprises the *Blautia hydrogenotrophica* bacterial strain as part of a microbial consortium.

7. The composition for use according to any one of claims 1-5, wherein the composition comprises only bacteria of the species *Blautia hydrogenotrophica.*

8. The composition for use according to any preceding claim, wherein the composition does not comprise bacteria of the genus *Clostridium.*

9. The composition for use according to any preceding claim, wherein the bacterial strain has a 16s rRNA sequence that is at least 95%, 96%, 97%, 98%, 99%, 99.5% or 99.9% identical to SEQ ID NO 5

10. The composition for use according to any preceding claim, wherein the composition is formulated as a probiotic.

11. The composition for use according to any preceding claim, wherein the composition comprises a prebiotic compound.

12. The composition for use according to claim 11, wherein the prebiotic compound is a non-digestible carbohydrate or a sugar alcohol.

13. The composition for use according to claim 12, wherein the non-digestible carbohydrate is selected from the group consisting of: fructo-oligosaccharides (or FOS), short-chain fructo-oligosaccharides, inulin, isomalt-oligosaccharides, pectins, xylo-oligosaccharides (or XOS), chitosan-oligosaccharides (or COS), beta-glucans, arable gum modified and resistant starches, polydextrose, D-tagatose, acacia fibers, carob, oats, and citrus fibers.

14. The compositions for use according to any preceding claim, wherein the composition comprises granules or gelatin capsules.

## Patentansprüche

1. Zusammensetzung, umfassend einen Bakterienstamm der Spezies *Blautia hydrogenotrophica,* zur Verwendung bei:
(a) einem Verfahren zur Behandlung oder Vorbeugung von sensorischer Überempfindlichkeit bei einem Individuum, bei dem Fibromyalgie diagnostiziert wurde; oder
(b) einem Verfahren zur Behandlung oder Vorbeugung von Allodynie und/oder Hyperalgesie, dies jeweils in Zusammenhang mit sensorischer Überempfindlichkeit bei einem Individuum, bei dem Fibromyalgie diagnostiziert wurde, stehen; oder
(c) einem Verfahren zur Behandlung oder Vorbeugung von Fibromyalgie.

2. Zusammensetzung zur Verwendung nach Anspruch 1, wobei die Zusammensetzung zur Verwendung bei einem Patienten vorgesehen ist, bei dem keine viszerale Überempfindlichkeit diagnostiziert wurde.

3. Zusammensetzung zur Verwendung nach einem vorhergehenden Anspruch, wobei die Zusammensetzung zur oralen Verabreichung vorgesehen ist.

4. Zusammensetzung zur Verwendung nach einem vorhergehenden Anspruch, wobei die Zusammensetzung einen oder mehrere pharmazeutisch unbedenkliche Exzipienten oder Träger umfasst.

5. Zusammensetzung zur Verwendung nach einem vorhergehenden Anspruch, wobei der Bakterienstamm lyophilisiert oder lebensfähig ist.

6. Zusammensetzung zur Verwendung nach einem vorhergehenden Anspruch, wobei die Zusammensetzung den Bakterienstamm *Blautia hydrogenotrophica* als Teil einer mikrobiellen Gemeinschaft umfasst.

7. Zusammensetzung zur Verwendung nach einem der Ansprüche 1-5, wobei die Zusammensetzung nur Bakterien der Spezies *Blautia hydrogenotrophica* umfasst.

8. Zusammensetzung zur Verwendung nach einem vorhergehenden Anspruch, wobei die Zusammensetzung keine Bakterien der Gattung *Clostridium* umfasst.

9. Zusammensetzung zur Verwendung nach einem vorhergehenden Anspruch, wobei der Bakterienstamm eine 16s-rRNA-Sequenz aufweist, die zu wenigstens 95%, 96%, 97%, 98%, 99%, 99,5% oder 99,9% mit SEQ ID NO:5 identisch ist.

10. Zusammensetzung zur Verwendung nach einem vorhergehenden Anspruch, wobei die Zusammensetzung als Probiotikum formuliert ist.

11. Zusammensetzung zur Verwendung nach einem vorhergehenden Anspruch, wobei die Zusammensetzung eine präbiotische Verbindung umfasst.

12. Zusammensetzung zur Verwendung nach Anspruch 11, wobei es sich bei der präbiotischen Verbindung um ein unverdauliches Kohlenhydrat oder einen Zuckeralkohol handelt.

13. Zusammensetzung zur Verwendung nach Anspruch 12, wobei das unverdauliche Kohlenhydrat ausgewählt ist aus der Gruppe bestehend aus: Fructo-Oligosacchariden (oder FOS), kurzkettigen Fructo-Oligosacchariden, Inulin, Isomalto-Oligosacchariden, Pektinen, Xylo-Oligosacchariden (oder XOS), Chitosan-Oligosacchariden (oder COS), Beta-Glucanen, mit Gummiarabikum modifizierten und resistenten Stärken, Polydextrose, D-Tagatose, Akazienfasern, Johannisbrot, Hafer und Zitrusfruchtfaser.

14. Zusammensetzungen zur Verwendung nach einem vorhergehenden Anspruch, wobei die Zusammensetzung Granulate oder Gelatinekapseln umfasst.

## Revendications

1. Composition comprenant une souche bactérienne de l'espèce *Blautia hydrogenotrophica* pour une utilisation dans :
(a) un procédé de traitement ou de prévention de l'hypersensibilité sensorielle chez un sujet chez lequel on a diagnostiqué une fibromyalgie ; ou
(b) un procédé de traitement ou de prévention de l'allodynie et/ou de l'hyperalgie qui sont toutes deux associées à l'hypersensibilité sensorielle chez un sujet chez lequel on a diagnostiqué une fibromyalgie ; ou
(c) un procédé de traitement ou de prévention de la fibromyalgie.

2. Composition pour une utilisation selon la revendication 1, la composition étant pour une utilisation chez un patient chez lequel une hypersensibilité viscérale n'a pas été diagnostiquée.

3. Composition pour une utilisation selon une quelconque revendication précédente, la composition étant pour une administration orale.

4. Composition pour une utilisation selon une quelconque revendication précédente, la composition comprenant un ou plusieurs excipients ou supports pharmaceutiquement acceptables.

5. Composition pour une utilisation selon une quelconque revendication précédente, la souche bactérienne étant lyophilisée ou viable.

6. Composition pour une utilisation selon une quelconque revendication précédente, la composition comprenant la souche bactérienne *Blautia hydrogenotrophica* comme une partie d'un consortium microbien.

7. Composition pour une utilisation selon l'une quelconque des revendications 1 à 5, la composition comprenant seulement des bactéries de l'espèce *Blautia hydrogenotrophica.*

8. Composition pour une utilisation selon une quelconque revendication précédente, la composition ne comprenant pas de bactéries du genre *Clostridium.*

9. Composition pour une utilisation selon une quelconque revendication précédente, la souche bactérienne possédant une séquence d'ARNr 16s qui est au moins 95 %, 96 %, 97 %, 98 %, 99 %, 99,5 % ou 99,9 % identique à la SEQ ID NO: 5.

10. Composition pour une utilisation selon une quelconque revendication précédente, la composition étant formulée comme un probiotique.

11. Composition pour une utilisation selon une quelconque revendication précédente, la composition comprenant un composé prébiotique.

12. Composition pour une utilisation selon la revendication 11, le composé prébiotique étant un glucide non digestible ou un alcool de sucre.

13. Composition pour une utilisation selon la revendication 12, le glucide non digestible étant choisi dans le groupe constitué par : des fructo-oligosaccharides (ou FOS), des fructo-oligosaccharides à chaîne courte, l'inuline, des isomalt-oligosaccharides, des pectines, des xylo-oligosaccharides (ou XOS), des chitosaneoligosaccharides (ou COS), des bêta-glucanes, des amidons modifiés par une gomme arable et résistants, un polydextrose, le D-tagatose, des fibres d'acacia, des fibres de caroube, d'avoine et d'agrumes.

14. Compositions pour une utilisation selon une quelconque revendication précédente, la composition comprenant des granules ou des capsules de gélatine.
